Europäisches Patentamt

European Patent Office

Office européen des brevets

(11) Publication number: **0 173 557**
**B1**

# EUROPEAN PATENT SPECIFICATION

(45) Date of publication of patent specification: **16.01.91**

(21) Application number: **85306085.3**

(22) Date of filing: **28.08.85**

(51) Int. Cl.⁵: **C 07 K 7/08,** C 07 K 7/10, A 61 K 37/02

(54) Peptides.

(30) Priority: **29.08.84 JP 180000/84**

(43) Date of publication of application:
**05.03.86 Bulletin 86/10**

(45) Publication of the grant of the patent:
**16.01.91 Bulletin 91/03**

(84) Designated Contracting States:
**CH DE FR GB IT LI**

(56) References cited:
**EP-A-0 140 731**
**EP-A-0 142 487**
**WO-A-85/04870**
**WO-A-85/04872**

**CHEMICAL ABSTRACTS, vol. 101, no. 5, 30th July 1984, page 66, abstract no. 33377k, Columbus, Ohio, US; D.M. GELLER et al.: "Atriopeptins: a family of potent biologically active peptides derived from mammalian atria", & BIOCHEM. BIOPHYS. RES. COMMUN. 1984, 120(2), 333-8**

(73) Proprietor: **AJINOMOTO CO., INC.**
**5-8, Kyobashi 1-chome, Chuo-ku**
**Tokyo 104 (JP)**

(72) Inventor: **Sakakibara, Shumpei**
**No. 2-23-2, Fujishirodai**
**Suita-shi Osaka-fu (JP)**

(74) Representative: **Bond, Bentley George et al**
**Haseltine Lake & Co. 28 Southampton Buildings**
**Chancery Lane**
**London WC2A 1AT (GB)**

(56) References cited:
**NATURE, vol. 309, 21st June 1984, pages 717-719, Macmillan Publishers, London, GB; S.A. ATLAS et al.: "Purification, sequencing and synthesis of natriuretic and vasoactive rat atrial peptide"**

**CHEMICAL ABSTRACTS, vol. 103, no. 23, 9th December 1985, page 711, abstract no. 196391r, Columbus, Ohio, US; T.M.**

Courier Press, Leamington Spa, England.

(56) References cited:

BALASUBRAMANIAN et al.: "Synthesis and biological activity of proposed structures for the atriopeptin family of naturetic hormones", & PEPT., PROC. EUR. PEPT. SYMP., 18th 1984, 509-12

BIOCHEMICAL AND BIOPHYSICAL RESEARCH COMMUNICATIONS, vol. 117, no. 3, 28th December 1983, pages 859-865, Academic Press, Inc., New York, US; T.G. FLYNN et al.: "The amino acid sequence of an atrial peptide with potent diuretic and natriuretic properties"

BIOCHEMICAL AND BIOPHYSICAL RESEARCH COMMUNICATIONS, vol. 118, no. 1, 13th January 1984, pages 131-139, Academic Press, Inc., New York, US; K. KANGAWA et al.: "Purification and complete amino acid sequence of alpha-human atrial natriuretic polypeptide (alpha-hANP)"

CHEMICAL ABSTRACTS, vol. 103, no. 9, 2nd September 1985, page 81, abstract no. 65114e, Columbus, Ohio, US; Y. HIRATA et al.: "Vascular receptor binding activities and cyclic GMP responses by synthetic human and rat atrial natriuretic peptides (ANP) and receptor down-regulation by ANP", & BIOCHEM. BIOPHYS. RES. COMMUN. 1985, 128(2) 538-46

Naoyoshi Chino et al., "Peptide chemistry 1984", edited by N. Izumiyos, pages 241-246

Pages 14, 45,46 and 48 of the catalogue issued in 1985 by Peninsular Laboratories

**Description**

This invention relates to peptides for use as drugs, for example for use as diuretic agents, for use as therapeutic agents for the treatment of hypertension, for use as therapeutic agents for the treatment of heart disease, and for use as muscle-relaxing agents.

It is known that 95% of hypertension is essential hypertension and that half thereof is a sodium-sensitive hypertension. It is possible to control this type of hypertension by controlling the amount of sodium in the living body. It has been thought that, in the factors relevant to natriuretic action, there is an unknown humoral factor (the third factor) in addition to glomerular filtration rate and ardestone. In the third factor, there is involved one substance which inhibits Na-K ATPase, and one substance which does not inhibit it. Therefore, it is to be expected that clarification of the above would give rise to an epoch-making state of affairs relative to the cause and treatment of essential hypertension.

Since the autumn of 1983, the establishment of the structure of natriuretic hormone as secreted from an atrium, one of the third factors (i.e. the one without Na-K ATPase inhibitory activity), has been reported many times. This substance possesses a strong natriuretic activity and a strong muscle-relaxing activity. One of the natriuretic peptide hormones as isolated from humans is α-human atrial natriuretic peptide (abbreviated as α-hANP) having the following structural formula:

$$H-Ser^1-Leu-Arg-Arg-Ser-Ser-Cys-Phe-Gly-Gly-Arg-Met-$$

$$-Asp-Arg-Ile-Gly-Ala-Gln-Ser-Gly-Leu-Gly-Cys-$$

$$-Asn-Ser-Phe-Arg-Tyr^{28}-OH$$

One of the natriuretic peptide hormones isolated from rat's atria is α-rat atrial natriuretic peptide (abbreviated as α-rANP), having the following structural formula:

$$H-Ser-Leu-Arg-Arg-Ser-Ser-Cys-Phe-Gly-Gly$$

$$-Arg-Ile-Asp-Arg-Ile-Gly-Ala-Gln-Ser-Gly-$$

$$-Leu-Gly-Cys-Asn-Ser-Phe-Arg-Tyr-OH$$

For a long time, diuretic anti-hypertensive drugs have been widely employed as a first choice drug for the treatment of patients with hypertension. However, recently, the side effects of such drugs, in causing heart disease, have been discovered and therefore, improvements in such drugs have been sought. Thus, the development of new drugs having less toxicity and more positive activity has been strongly desired by clinicians.

The compound α-hANP is an endogeneous factor and a peptide, and therefor the safety thereof is presumed to be very high. However, on the other hand, because the fact that it is peptide, there are thought to be many problems in its developing as a drug, which problems include the fact that it undergoes decomposition with peptidase, the fact that the duration of its activity is short, and the fact that it has an unstable nature.

The inventors of the present invention have devised two peptides related to α-hANP, for use as drugs for peptide circulating systems.

The two peptides of the present invention are as follows:

## α-hANP(7-28)

```
┌─────────────────────────────────────────────────────────────────┐
  H-Cys-Phe-Gly-Gly-Arg-Met-Asp-Arg-Ile-Gly-Ala-Gln-              │
                          ┌────────────────────────────────────────┘
  Ser-Gly-Leu-Gly-Cys-Asn-Ser-Phe-Arg-Tyr-OH
```

## (Asu$^{7,23}$) - α-hANP(7-23)

```
┌─Phe-Gly-Gly-Arg-Met-Asp-Arg-Ile-Gly-
│
│
└─────────────────────────────────────┐
  Ala-Gln-Ser-Gly-Leu-Gly-Asu-OH
```

The amino acids constituting the peptide derivatives of the present invention may be L-isomers or D-isomers.

The peptides of the present invention may be in the form of a salt, such as a metal salt (for example a sodium, potassium, lithium or calcium salt) or a salt with an organic base. As the organic base, there can be used amines such as ammonia (ammonium salt), dicyclohexylamine, and N-methyl-D-glucamine, and basic amino acids such as lysine and arginine.

The peptides of the present invention may be in the form of a salt with a mineral acid (such as hydrochloric acid, sulphuric acid or phosphoric acid) or with an organic acid (such as acetic acid or maleic acid).

When the peptides of the present invention possess one or more functional groups (such as amino groups, carboxyl groups, hydroxyl groups or guanidyl groups), a part or all of the functional groups may be protected by a protecting group used in methods for peptide synthesis or generally or conventionally employed in the known literatures. These derivatives are included in the peptides of the present invention. For the methods for the protection thereof and the methods for removal of the protecting groups, conventional means for peptide synthesis may be employed.

When the peptides are included in drugs such as diuretics, they are in a form of a pharmaceutically acceptable salt or in free form. In the case of protected derivatives, these derivatives should be non-toxic.

The peptides of the present invention can be produced on the basis of the examples given below (wherein Examples 5 and 6 illustrate the preparation of peptides of the invention and the remaining Examples illustrate the preparation of related peptides) and on the basis of methods conventionally employed peptide synthesis, and the known literature, e.g. as disclosed in Protein Chemistry, 1, Amino acid, Peptide, published by Kyoritsu Shuppan Co. (1969).

As is apparent from the examples given below, the peptides of the present invention are useful as drugs for the circulatory system, for example as diuretics and as drugs for the treatment of heart disease.

The abbreviations used herein are as follows:

(1) Amino acid residues

Phe: phenylalanine; Gly: glycine; Arg: arginine; Asp: asparatic acid; Ile: isoleucine; Ala: alanine; Gln: glutamine; Ser: serine; Leu: leucine; Met: methionine; Met(O): methionine oxide; Nle: norleucine; Cys: cysteine; Asu: α-aminosuberic acid; Asn: asparagine; Tyr: tyrosine; Cys̶ ̶ ̶Cys: cystine; Glu: glutamic acid.

(2) Protecting groups

Boc: t-butyloxy carbonyl; 4-CH₃Bzl: p-methylbenzyl; Bzl: benzyl; Tos: tosyl; Cl₂Bzl: 2,6-dichlorobenzyl; Et: ethyl; Me: methyl; Pac: phenacyl; Su: succinimide; Chx: cyclohexyl.

(3) Reagents

DMF: dimethylformamide; AcOEt: ethyl acetate; TFA: trifluoroacetic acid; Et₂O: ether; HOBt: 1-hydroxybenzotriazole; CH₂Cl₂: dichloromethane; WSCI: water soluble carbodiimide; Ca: calcium; AcOH: acetic acid; HCl: hydrogen chloride or hydrochloric acid; TFE: trifluoroethane; NaHCO₃: sodium bicarbonate; n-hexane: n-hexane; TsOH: p-toluene sulphonic acid; HF: hydrogen fluoride; NaOH: sodium hydroxide; NEt₃: triethyl amine; MgSO₄: magnesium sulphate; MeOH: methanol, CHCl₃: chloroform; Zn:

zinc; NMP: N-methyl-2-pyrolidone; $P_2O_5$: phosphorus pentoxide; $CH_3CN$: acetonitrile; $Na_2SO_4$: sodium sulphate.

(4) Other abbreviations

mmole: Millimole; eq: equivalent; HPLC: High Performance Liquid Chromatography; Pd-C: palladium-carbon.

Example 1
Synthesis of α-hANP(5—27)
Synthesis of Boc-Cys(MeBzl)-Asn-Ser(Bzl)-Phe-Arg(Tos)-OBzl

(1) Synthesis of Boc-Phe-Arg(Tos)-OBzl

Boc-Phe-OH (4g, 15 mmole), H-Arg-OBzl.TosOH (8.9 g, 15 mmole) and HOBt (2.1 g, 15.75 mmole) were dissolved in DMF (25 ml), and WSCI (12.9 ml, 15.75 mmole) was added dropwise thereto while cooling, and the thus obtained mixture (pH = 4) was stirred overnight. To the reaction solution, AcOEt (300 ml) was added and the solution was washed with 1N HCl, water and 5% $NaHCO_3$ and water in the order given, and dried with $NaSO_4$. AcOEt was distilled off under reduced pressure, and the residue thus obtained was treated with ethyl acetate, ether and n-hexane to give an oily substance. The substance was recovered by decantation and treated with ethyl acetate and hexane to give the required product in powder form (6.89 g, 69%).

(2) Synthesis of Boc-Ser(Bzl)-Phe-Arg(Tos)-OBzl

A mixture of Boc-Phe-Arg(Tos)-OBzl (6.79 g, 10.2 mmole) and TFA (20 ml) was stirred for 10 minutes with cooling and then for 20 minutes at room temperature. TFA was distilled off under reduced pressure, and to the residue thus obtained, ether was added to precipitate a powder. The powder was dried under reduced pressure for 2 hours over NaOH, then dissolved in DMF (25 ml), and $NEt_3$ (1 ml) was added thereto while cooling. Next, the mixture was reacted with Boc-Ser-(Bzl)-OSu (4.2 g, 10.7 mmole) for 2.5 hours. After that, $NEt_3$ [0.4 ml (total volume: 1.4 ml, 10.2 mmole)] was added thereto, and the mixture was stirred for a further 40 hours with the addition of a small amount of $NEt_3$.

AcOEt (250 ml) was added to the solution as obtained above, and the solution was washed with 1N HCl, water, 5% $NaHCO_3$, and water, and dried with $MgSO_4$. AcOEt was distilled off from the solution, and the residue thus obtained was washed several times with AcOEt/ether and n-hexane by decantation. Thereafter, by using the same steps as above, the required product (7.7 g, 89.5%) was obtained in powder form.

(3) Synthesis of Boc-Asn-Ser(Bzl)-Phe-Arg(Tos)-OBzl

A mixture of Boc-Ser(Bzl)-Phe-Arg(Tos)]-OBzl (7.6 g, 9 mmole) and TFA (25 ml) was stirred for 10 minutes with cooling and then for 30 minutes at room temperature, and 6.9 N HCl/dioxane (1.6 ml, 10.8 mmole) was added thereto. The solvent was distilled off under reduced pressure. To the residue thus obtained, ether was added to give a powder, and the powder was recovered by filtration, and dried under reduced pressure for 3 hours over NaOH. The powder, together with Boc-Asn (2.3 g, 9.9 mmole) and HOBt (1.4 g, 9.9 mmole), was dissolved in DMF (25 ml), and WSCI (1.8 ml, 9.9 mmole) was added dropwise thereto with cooling. The mixture thus obtained (pH = 4.5) was stirred overnight, mixed with ethyl acetate (200 ml), washed with 1N HCl, water, 5% $NaHCO_3$ and water, and dried over $MgSO_4$. Ethyl acetate was distilled off therefrom, and the residue was washed twice with ethyl acetate and methanol/ether to obtain the required product (79.3 g, 84.9%) in powder form.

(4) Synthesis of Boc-Cys(4-MeBzl)-Asn-Ser(Bzl)-Phe-Arg(Tos)-OBzl

A mixture of Boc-Asn-Ser(Bzl)-Phe-Arg(Tos)-OBzl (3.8 g, 4 mmole) and TFA (15 ml) was stirred for 10 minutes with cooling and for 25 minutes at room temperature, and then 6.9 N HCl/dioxane (0.7 ml, 4.8 mmole) was added thereto. The solvent was distilled off therefrom, and to the residue thus obtained, $Et_2O$ was added. The powder thus precipitated was recovered by filtration, and dried for 3.5 hours over NaOH. The powder, together with Boc-Cys (4-MeBzl)-OH (1.43 g, 4.4 mmole) and HOBt (5.95 mg, 4.4 mmole), was dissolved in DMF (15 ml), and WSCI (0.81 ml, 4.4 mmole), was added dropwise thereto with cooling. The mixture (pH = 4.5) was stirred overnight, mixed with ethyl acetate (150 ml), washed with 1N HCl, water, 5% $NaHCO_3$ and water, dehydrated by toluene flashing, and treated twice with $CHCl_3$ and methanol/ether to obtain the required product (4.21 g, 90.3%) in powder form.

Amino acid analysis [Hydrolysis with 6N HCl, 108°C 22 hours, in the presence of phenol]

$NH_3$: 1.15,   Arg: 0.97,   Asp: 1.00,   Ser: 0.88,   Cys: small peak,   Phe: 1.00

Elementary analysis

Found        C 60.47%,   H 6.37%,   N 10.76%,
Calculated   C 60.39%,   H 6.36%,   N 10.74%
(for $C_{57}H_{73}O_{12}N_9S_2 \cdot 1/2H_2O$)

Synthesis of Boc-Ala-Gln-Ser(Bzl)-Gly-Leu-Gly-OH

(5) Synthesis of Boc-Ser(Bzl)-Gly-OH

Box-Ser(Bzl)-OH (14.8 g, 50 mmole) and H-Gly-OEt HCl (8.4 g, 60 mmole) were suspended in $CH_2Cl_2$ (100 ml), WSCI (10 ml, 55 mmole) was added dropwise thereto and the mixture (pH = 7) was stirred overnight.

$CH_2Cl_2$ was distilled off under reduced pressure, and the residue thus obtained was dissolved in ether (300 ml) and water (50 ml). The solution thus obtained was washed with 1N HCl, water, 5% $NaHCO_3$ and water, and then the ether was distilled off therefrom. The oily substance thus obtained was dissolved in MeOH (50 ml), 1N HaOH (50 ml, 50 mmole) was added dropwise thereto with cooling at a temperature of 0 to 1°C, and soon after removal of the refrigerant, the solution was stirred for 60 minutes.

The solution was cooled again and adjusted to pH 7 with 6N HCl, and methanol was distilled off. The residue thus obtained was dissolved in aqueous $NaHCO_3$, washed twice with ether, and the aqueous phase thereof was adjusted to pH 2 with 6N HCl. The mixture thus obtained was extracted with ethyl acetate (250 ml), washed with water and dried over anhydrous sodium sulphate. Ethyl acetate was distilled off therefrom under reduced pressure to obtain quantitatively the required product in oily form.

(6) Synthesis of Boc-Ser(Bzl)-Gly-Leu-Gly-OPac

A mixture of Boc-Leu-Gly-OPac (16.3 g, 40 mmole) and TFA (40 ml) was stirred for 10 minutes with cooling and then for 30 minutes at room temperature, and 3.5N HCl in dioxane (13.7 ml, 48 mmole) was added thereto. The solvent was distilled off therefrom and to the residue, ether-n-hexane was added to give an oily substance. The solvent was decanted and the residual substance was pulverized with ether-n-hexane. The powder thus obtained was recovered by filtration and then dried for 3.5 hours over sodium hydroxide. The powder together with Boc-Ser(Bzl)Gly-OH (total volume of the above produced oily substance) and HOBt (5.9 g, 44 mmole), was dissolved in DMF (60 ml), and WSCI (18 ml, 44 mmole) was added dropwise thereto with cooling. The solution (pH = 4) was stirred overnight, mixed with ethyl acetate (400 ml), washed with 1N HCl, water, 5% aqueous $NaHCO_3$ and water, and dried over anhydrous sodium sulphate. AcOEt was distilled off therefrom, and the residue thus obtained was treated twice with AcOEt/$Et_2O$ to obtain a gel-like powder of the required product (19.3 g, 75.4%).

Amino acid analysis [Hydrolysis with 6N HCl]

Ser: 0.91, Gly: 1.00 × 2, Leu: 1.02

(7) Synthesis of Boc-Gln-Ser(Bzl)-Gly-Leu-Gly-OPac

Boc-Ser(Bzl)-Gly-Leu-Gly-Opac (19.2 g, 30 mmole) was reacted with TFA (50 ml) for 10 minutes with cooling and then for 40 minutes at room temperature, and 3.5N HCl in dioxane (10.3 ml, 36 mmole) was added thereto. The solvent was distilled off therefrom and to the residue thus obtained, ether-n-hexane was added. The powder thus obtained was recovered by filtration and dried for 3 hours over NaOH. The powder, together with Boc-Gln-OH (8.1 g, 33 mmole) and HOBt (4.7 g, 34.5 mmole), was dissolved in DMF (50 ml), WSCI (6.3 ml, 34.5 mmole) was added dropwise thereto with cooling and the solution (pH = 4) was stirred overnight to give a solid. Water was added thereto with cooling and the powder thus obtained was recovered by filtration, and washed with water, n-hexane and ether. The powder thus obtained was dissolved in warm chloroform/methanol, and then dehydrated by flashing with toluene. Chloroform/methanol was added thereto and the suspension thus obtained was treated twice with ether with heating to obtain the required product (22.3 g, 96.5%).

(8) Synthesis of Boc-Ala-Gln-Ser(Bzl)-Gly-Leu-Gly-OPac

Boc-Gln-Ser(Bzl)-Gly-Leu-Gly-Opac (11.5 g, 15 mmole) was reacted with TFA (30 ml) for 10 minutes with cooling and then for 45 minutes at room temperature, and 6.9N HCl in dioxane (2.6 ml, 18 mmole) was added thereto. The solvent was distilled off therefrom and to the residue thus obtained, ether was added. The powder thus obtained was recovered by filtration and dried for 3 hours over NaOH. The powder, together wih Boc-Ala (3.1 g, 16.5 mmole) and HOBt (2.2 g, 16.5 mmole), was dissolved in DMF (30 ml), WSCI (3 ml, 16.5 mmole) was added dropwise thereto with cooling to effect reaction (pH = 5). After about 30 minutes, a gel was formed, which gel was mixed with DMF (30 ml) and then stirred overnight.

Water was added thereto with cooling and the powder thus obtained was recovered by filtration, and washed with water, n-hexane and ether, dissolved in $CHCl_3$ and methanol, dehydrated by flashing with toluene, and purified by heating twice with $CHCl_3$-methanol/ether to obtain the required product (11.65 g 92.5%).

Amino acid analysis [Hydrolysis with 6N HCl]

$NH_3$: 1.10, Ser: 0.93, Gln: 1.00, Gly: 1.02 × 2, Ala: 1.00, Leu: 1.01

Elementary analysis

Found        C 58.14%,  H 6.92%,  N 11.57%

Calculated  C 58.01%,  H 6.89%,  N 11.55%

(for $C_{41}H_{57}O_{12}N_7 \cdot 1/2H_2O$)

(9) Synthesis of Boc-Ala-Gln-Ser(Bzl)-Gly-Leu-Gly-OH

Boc-Ala-Gln-Ser(Bzl)-Gly-Leu-Gly-OPac (10.9 g, 13 mmole) was dissolved in acetic acid (100 ml) with heating, and then cooled naturally. Powdered zinc (17 g) was added thereto and the mixture thus obtained was stirred for 50 minutes on a water bath at 43°C. The powdered zinc was removed by filtration and acetic acid was distilled off. To the residue thus obtained, water was added to give a powder. The powder was recovered by filtration and washed with water. This powder is called "Powder A". The mother liquor as produced above gave a powder by the addition of n-hexane, and the powder was recovered by filtration and washed with ether. This powder is called "Powder B". Powder A and powder B were combined together and dissolved in heated methanol, dehydrated by flashing with toluene, and gave the required product (8.93 g, 95%) by reprecipitation from heated methanol/ether.

Amino acid analysis [Hydrolysis with 6N HCl]

NH$_3$: 1.16, Ser: 0.91, Glu: 1.00, Gly: 1.03 × 2, Ala: 1.00, Leu: 1.05

Elementary analysis

Found     C 54.45%,   H 7.18%,   N 13.36%

Calculated   C 54.50%,   H 7.15%,   N 13.48%

(as $C_{33}H_{51}O_{11}N_7 \cdot 0.3H_2O$)

(10) Synthesis of Boc-Ala-Gln-Se(Bzl)-Gly-Leu-Gly-Cys(4-MeBzl)-Asn-Se(Bzl)-Phe-Arg(Tos)-OBzl

Boc-Cys(4-MeBzl)-Asn-Gln-Ser(Bzl)-Gly-Leu-Gly-Cys(4-MeBzl)-Asn-Ser(Bzl)-Phe-Arg(Tos-OBzl

Boc-Cys(4-MeBzl)-Asn-Ser(Bzl)-Phe-Arg(Tos)-OBzl (1.63 g, 1.4 mmole) was reacted with TFA (5 ml) for 10 minutes with cooling and then for 50 minutes at room temperature, and 3.5 N HCl in dioxane (0.48 ml, 1.68 mmole) was added thereto. The solvent was distilled off therefrom and the residue thus obtained gave a powder by the addition of ether. The powder was recovered by filtration and dried for 3 hours over NaOH.

The powder, together with Boc-Ala-Gln-Ser(Bzl)-Gly-Leu-Gly-OH (1.06 g, 1.47 mmole) and HOBt (208 mg, 1.54 mmole), was dissolved in DMF (30 ml), WSCI (0.28 ml, 1.54 mmole) was added dropwise thereto with cooling. The solution thus obtained (pH = 4.5) was stirred overnight and then solidified. Water was added thereto with cooling. The powder thus produced was recovered by filtration, and washed with water, 5% aqueous NaHCO$_3$, water, n-hexane and ether in the order given, and dried over P$_2$O$_5$. The substance was dissolved in heated DMF (250 ml), and insoluble matter was removed by filtration. The DMF was distilled off and the residue thus obtained gave the required product (2.33 g, 94%) by reprecipitation from heated DMF/methanol.

Amino acid analysis [Hydrolysis with 6N HCl]

Arg: 0.95, Asp: 1.00, Ser: 0.84 × 2, Glu: 0.95, Gly: 1.00 × 2, Ala: 0.98, Cys: small peak, Leu: 1.04, Phe: 0.99

Elementary analysis

Found     C 58.92%,   H 6.60%,   N 12.55%

Calculated   C 59.10%,   H 6.5%,   N 12.68%

(for $C_{87}H_{114}O_{20}N_{16}S_2$)

(11) Synthesis of Boc-Ile-Gly-OEt

Gly-OEt-HCl (9.77 g, 70.0 mmole), Boc-Ile.1/2H$_2$O (17.7 g, 73.5 mmole) (which had been previously dissolved in CHCl$_3$ and toluene, concentrated and dehydrated), and HOBt (9.93 g, 73.5 mmole) were dissolved in DMF (70 ml), and WSCI (13.5 ml, 73.5 mmole) were added dropwise thereto while cooling and stirring. Next day, it was confirmed that a fluorolescamine test was negative. To the reaction solution, water was added and the thus separated oily substance was recovered, by extraction with ethyl acetate. The ethyl acetate phase was washed with 5% aqueous NaHCO$_3$, 1N HCl and aqueous NaCl in the order given and dried over MgSO$_4$. The ethyl acetate was distilled off and the crystalline residue thus obtained was recrystallized from ethyl acetate/n-hexane to give the required product (17.5 g, 79%) in the form of crystals.

(12) Synthesis of Boc-Ile-Gly-OH

Boc-Ile-Gly-OEt (17.4 g, 55.5 mmole) was dissolved in methanol (60 ml) and 2N NaOH (33 ml, 66.6 mmole) was added dropwise thereto while cooling and stirring. The cooling bath was removed, and the solution was stirred for 1 hour at about 15°C, and then neutralized with 6N HCl. Methanol was distilled off, and the solution was adjusted to a pH of about 2 and extracted with ethyl acetate. The ethyl acetate layer thus obtained was washed with water and dried over MgSO$_4$. Ethyl acetate was distilled off therefrom, and the thus obtained oily residue was crystallized from ethyl acetate/n-hexane to give the required product (15.5 g, 98%).

(13) Synthesis of Boc-Ile-Gly-OPac

Boc-Ile-Gly-OH (15.3 g, 53.1 mmole) and Pac-Br (10.8 g, 54.2 mmole) were dissolved in DMF (50 ml) and Et$_3$N (7.4 ml, 54.2 mmole) was added dropwise thereto with cooling. Four hours later, water was poured into the reaction solution and the mixture thus obtained was extracted with ethyl acetate. The ethyl acetate layer was washed with 1N HCl, water, 5% aqueous NaHCO$_3$, and water in the given order and dried over MgSO$_4$. Ethyl acetate was distilled off, and the residue thus obtained was recrystallized from methanol/ether. The yield was 16.0 g.

The mother liquor produced above was concentrated and gave the required product (5.4 g) by recrystallization from ethyl acetate/n-hexane. Both of the above portions of crystals were combined to give a yield of 21.4 g (99%).

(14) Synthesis of Boc-Arg(Tos)-Ile-Gly-OPac

Boc-Ile-Gly-OPac (14.3 g, 35.18 mmole) was treated with $CF_3CO_2H$ (80 ml) for 10 minutes with cooling and for 50 minutes at room temperature, and 3.5N HCl in dioxane (12.1 ml, 42.2 mmole) was added dropwise thereto. The excess acid was distilled off. The residue was crystallized by the addition of ether and n-hexane, and the crystals thus obtained were dried over NaOH. These crystals, Boc-Arg (Tos) (15.9 g, 36.9 mmole) and HOBt (5.0 g, 36.9 mmole), were dissolved in DMF (50 ml), and WSCI (16.76 ml), 36.9 mmole) was added dropwise thereto while cooling and stirring. The solution was adjusted to a pH of about 5 by the addition of $Et_3N$. On the next day, Boc-Arg (Tos) (1.50 g), HOBt (0.48 g) and WSCI (0.66 ml), each corresponding to 0.1 eg, were further added thereto. On the following day, it was confirmed that a fluorolescamine test was negative. Water was poured into the reaction solution and the separated oily substance was obtained by extraction with ethyl acetate. The ethyl acetate phase was washed with 5% aqueous $NaHCO_3$, 1N HCl and water in the order given. When the precipitation of a gell-like material started, ethyl acetate was distilled off. Ether and n-hexane were added to the residue obtained to give a precipitate. The precipitate was recovered by filtration, dried and recrystallized from methanol/ether to give a yield of 22.0 g (87%).

(15) Synthesis of Boc-Asp-(OBzl)-Arg-Ile-Gly-Opac

Boc-Arg(Tos)-Ile-Gly-OPac (21.8 g, 30.4 mmole) was treated with $CF_3CO_2H$ (90 ml) for 10 minutes with cooling and for 50 minutes at room temperature. 6.9N HCl in dioxane (5.3 ml, 36.48 mmole) was added thereto and excess acid was distilled off. Ether was added thereto to give a powder, and the powder was dried over NaOH. The powder, together with Boc-Asp (OBzl) (10.3 g, 31.9 mmole) and HOBt (4.31 g, 31.9 mmole), were dissolved in DMF (60 ml), and WSCI (5.84 ml, 31.9 mmole) was added dropwise thereto while cooling and stirring. The solution thus obtained was adjusted to pH 4 to 5 by the addition of $Et_3N$. On the following day, Boc-Asp (OBzl) (0.30 g, 0.05 eq), HOBt (0.15 g, 0.05 eq) and WSCI (10.28 ml, 0.05 eq) were further added thereto. After 2 hours, it was confirmed that a fluorolescamine test was negative. Water was poured into the mixture and the mixture thus obtained was extracted with ethyl acetate. The ethyl acetate layer was washed with 5% aqueous $NaHCO_3$, water and aqueous saturated NaCl in the order given and dried over $MgSO_4$. Ethyl acetate was distilled off and the solid residue thus obtained was crystallized from methanol ether to give a yield of 27.6 g (98%).

(16) Synthesis of Boc-Met-Asp(OBzl)-Arg(Tos)-Ile-Gly-OPac

Boc-Asp(OBzl)-Arg(Tos)-Ile-Gly-OPac (27.5 g, 29.8 mmole) was reacted with $CF_3CO_2H$ (110 ml) for 10 minutes with cooling and for 50 minutes at room temperature, and 6.9N HCl/dioxane (5.2 ml, 35.8 mmole) was added thereto. Excess acid was distilled off therefrom. The powder as obtained by the addition of ether was dried over NaOH. The powder, corresponding to 25.0 mmole, among all the powders as produced above, Boc-Met (6.54 g, 26.3 mmole) and HOBt (3.55 g, 26.3 mmole), were dissolved in DMF (40 ml), and WSCI (4.80 ml, 26.3 mmole) was added dropwise thereto while cooling and stirring (pH about 5). On the following day, it was confirmed that a fluorolescamine test was negative. Water and ethyl acetate were poured into the reaction solution and the gell-like substance thus precipitated was recovered by filtration, washed with ether and reprecipitated from methanol/ether to give a yield of 25.0 g (95%).

(17) Synthesis of Boc-Arg(Tos)-Met-Asp(OBzl)-Arg(Tos)-Ile-Gly-OPac

Boc-Met-Asp-(OBzl)-Arg(Tos)-Ile-Gly-OPac (24.5 g, 23.3 mmole) was treated with $CF_3CO_2H$ (100 ml) for 10 minutes with cooling and for 50 minutes at room temperature, and 6.9N HCl in dioxane (4.1 ml, 28 mmole) was added thereto and excess acid was distilled off. Ether was added to give a powder, and the powder was recovered by filtration, and dried over NaOH. The above powder, together with Boc-Arg (Tos) (11.0 g, 25.6 mmole) and HOBt (3.5 g, 25.6 mmole), was dissolved in DMF (60 ml), and WSCI (4.69 ml, 25.6 mmole) was added dropwise thereto while cooling and stirring (pH of about 5). On the following day, it was confirmed that it had a negative response in a fluorolescamine test. Water was added thereto, and the solid thus precipitated by filtration, and washed with water and then ether. By reprecipitation from chloroform-methanol/ether, the required product was obtained in a yield of 29.8 g (94%).

(18) Synthesis of Boc-Arg(Tos)-Met-Asp(OBzl)-Ar(Tos)Ile-Gly-OH

Boc-Arg(Tos)-Met-Asp(OBzl)-Arg(Tos)-Ile-Gly-Opac (13.6 g, 10.0 mmole) was dissolved in acetic acid (50 ml), powdered zinc (14 g) was added thereto and the mixture thus obtained was heated to 47 to 48°C. After 1 hour, the reaction was completed. The catalyst was removed by filtration and then acetic acid was distilled off therefrom. Water was added and the solid substance thus precipitated was recovered by filtration, and washed with water and then ether. It was reprecipitated twice from chloroform-methanol/ether whereby the required product was obtained (11.98 g, 96%).

8

(19) Synthesis of Boc-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-OBzl

The peptide (2.2 g, 1.25 mmole) obtained in the above preparation (10) was treated with TFA (10 ml) for 10 minutes with cooling and for 45 minutes at room temperature, and 3.5N HCl/dioxane (0.43 ml, 1.5 mmole) was added thereto. Thereafter, the solvent was distilled off therefrom and to the residue thus obtained, ether was added and the powder thus obtained was recovered by filtration and dried overnight over NaOH.

The above powder, together with Boc-Arg(Tos)-Met-Asp-Arg(Tos)-Ile-Gly-OH (1.6 g, 1.29 mmole) and HOBt (177 mg, 1.31 mmole) were dissolved in a solvent produced by mixing DMF (15 ml) and NMP (10 ml), and WSCI (0.24 ml, 1.31 mmole) was added thereto with cooling and allowed to react. After 3 hours, it gelatinized in all, and a further amount of NMP (10 ml) was added. After stirring for one night, it had a negative response in a fluorescamine test. Water was added thereto with cooling to give a powder. The powdered substance thus produced was recovered by filtration, washed with water, n-hexane and ether, and dried over $P_2O_5$. The substance was dissolved repeatedly with heated DMF, whose total volume was 300 ml, to remove insoluble impurities by filtration. The DMF was distilled off and the residue thus obtained was treated with heated DMF/methanol to give the required product (3.3 g, 91.2%) in powder form.

(20) Synthesis of Boc-Gly-Gly-OPac

Boc-Gly-Gly-OH (9.28 g, 40 mmole) and phenacyl bromide (8.76 g, 44 mmole) were dissolved in DMF (40 ml) and $Et_3N$ (6.16 ml, 44 mmole) was added thereto with cooling. After 5 hours, water was added to the reaction solution and the thus precipitated substance was removed by extraction with ethyl acetate. The ethyl acetate layer was washed with 1N HCl, 5% aqueous $NaHCO_3$, and water in the order given, and dried over $MgSO_4$. The ethyl acetate was distilled off therefrom and the residue thus obtained was recrystallized from ethyl acetate-n-hexane to give a yield of 13.0 g (92.9%).

(21) Synthesis of Boc-Phe-Gly-Gly-OPac

Boc-Gly-Gly-OPac (12.6 g, 36 mmole) and TFA (50 ml) were mixed together and stirred for 45 minutes. The TFA was distilled off therefrom, and to the residue thus obtained, 0.9N HCl/dioxane (7.8 ml, 54 mmole) was added and then mixed well. Ether was added, and the thus precipitated substance was recovered by filtration, dried, and suspended in DMF (80 ml), and HOBt (5.35 g, 39.6 mmole), Boc-Phe-OH (10.5 g, 39.6 mmole) and WSCI (7.25 ml, 39.6 mmole) were added thereto with cooling to −15°C. After 16 hours of stirring, water was added to the reaction solution, and the precipitated substance was recovered by filtration and recrystallized form methanol to give a yield of 15.3 g (85.5%).

(22) Synthesis of Boc-Cys(4-MeBzl)-Phe-Gly-Gly-OPac

To Boc-Phe-Gly-Gly-OPac (12.4 g, 25 mmole), TFA (50 ml) was added and the mixture was stirred for 50 minutes. TFA was distilled therefrom and to the residue thus obtained, 3.5N HCl/dioxane (10.6 ml, 3.7 mmole) was added and stirred well, and then ether was added thereto. The precipitate produced was recovered by filtration, dried and dissolved in DMF (40 ml). HOBt (3.78 g, 28 mmole), Boc-Cys (4-MeBzl)-OH (9.1 g, 28 mmole) and WSCI (5.1 ml, 28 mmole) were added to the above solution with cooling to −15°C. After 16 hours of stirring, water was added to the reaction solution. The substance thus precipitated was recovered by filtration and then recrystallized twice from methanol to give a yield of 14.1 g (80.1%).

Amino acid analysis [Hydrolysis with 6N HCl]
    Arg: 0.98 × 3,  Asp: 1.00 × 2,  Ser: 0.88 × 2,  Glu: 0.95,  Gly: 1.01 × 3, Ala: 0.96,  Cys: small peak,  Met: 0.80,  Ile: 0.92,  Leu: 0.97,  Phe: 1.00.
Elementary analysis
    Found        C 56.27%,  H 6.48%,  N 13.40%
    Calculated   C 56.30%,  H 6.45%,  N 13.42%

(23) Synthesis of Boc-Ser(Bzl)-Cys(4-MeBzl)-Phe-Gly-Gly-OPac

To Boc-Cys(4-MeBzl)-Phe-Gly-Gly-OPac (12 g, 17 mmole), TFA (50 ml) was added and the mixture was stirred for 50 minutes. TFA was distilled off therefrom and to the residue thus obtained, 3.5N HCl/dioxane (7.3 ml, 25.5 mmole) was added and mixed well. Ether was added thereto and the substance thus precipitated was recovered by filtration, dried, and then dissolved in DMF (50 ml). HOBt (2.7 g, 20 mmole), Boc-Ser(Bzl)-OH (5.9 g, 20 mmole) and WSCI (37 ml, 20 mmole) were added to the above solution with cooling to −15°C. After 16 hours of stirring, water was added to the reaction solution and the precipitate thus obtained was recovered by filtration, and recrystallized twice from methanol to give a yield of 12.1 g (80.7%).

(24) Synthesis of Boc-Ser(Bzl)-Ser(Bzl)-Cys(4-MeBzl)-Phe-Gly-Gly-OPac

To Boc-Ser(Bzl)-Cys(4-MeBzl)-Phe-Gly-Gly-OPac (10 g, 11.3 mmole), TFA (40 ml) was added and the mixture was stirred for 50 minutes. TFA was distilled off, and to the residue thus obtained, 3.5N HCl/dioxane (4.8 ml, 17 mmole) was added and the mixture was stirred well, and then ether was added thereto. The substance thus precipitated was recovered by filtration, dried, and dissolved in DMF (30 ml). HOBt (1.69 g, 12.5 mmole), Boc-Ser(Bzl)-OH (3.7 g, 12.5 mmole) and WSCI (2.3 ml, 12.5 mmole) were added to the above solution with cooling to −15°C. After 16 hours of stirring, water was added to the reaction solution and the precipitated substance was recovered by filtration, and recrystallized twice from methanol to give a yield of 11.0 g (91.7%).

(25) Synthesis of Boc-Ser(Bzl)-Ser(Bzl)-Cys(4-MeBzl)-Phe-Gly-Gly-OH

Boc-Ser(Bzl)-Ser-(Bzl)-Cys(4-MeBzl)-Phe-Gly-Gly-OPac (8.25 g, 7.8 mmole) was dissolved in acetic acid (200 ml), powdered zinc (15 g) was added thereto and the mixture was stirred for 1 hour at 45°C. The zinc was removed by filtration and the acetic acid was concentrated, and to the residue thus obtained, water was added. The precipitate thus produced was recovered by filtration, dried and reprecipitated from chloroform/ether to give a yield of 7.0 g (95.5%).

Amino acid analysis [Hydrolysis with 6N HCl]

Ser: $0.93 \times 2$,   Gly: $1.02 \times 2$,   Phe: 1.00, 1/2 $(Cys)_2$:   Small peak

(26) Synthesis of Boc-Ser(Bzl)-Ser(Bzl)-Cys(4-MeBzl)-Phe-Gly-Gly-Arg(Tos)-Met-Asp(OBzl)Arg(Tos)-Ile-Gly-Ala-Gln-Ser(Bzl)-Gly-Leu-Gly-Cys(4-MeBzl)-Asn-Ser(Bzl)-Phe-Arg(Tos)-OBzl

Boc-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-OBzl (2.17 g, 0.75 mmole) was treated with TFA (10 ml) for 10 minutes with cooling and for 50 minutes at room temperature, and 3.5N HCl/dioxane (0.26 ml, 0.9 mmole) was added thereto. The solvent was distilled off and to the residue, ether was added and the powder thus produced was recovered by filtration and dried overnight over NaOH. The powder, together with Boc-Ser-(Bzl)-Ser(Bzl)-Cys(4-MeBzl)-Phe-Gly-Gly-OH (741 mg, 0.788 mmole) and HOBt (111 mg, 0.825 mmole) were dissolved in NMP (30 ml), and WSCI (150 ul, 0.825 mmole) was added thereto with cooling to allow reaction to take place (pH = 5). After 2 hours, the mixture gelatinized in all, and then NMP (10 ml) was added thereto and stirred overnight. Water was added thereto with cooling to give a powder and the powder thus obtained, was recovered by filtration, washed with water, n-hexane and ether in the order given, and dried over $P_2O_5$. By repeatedly dissolving the powder in heated NMP (140 ml) and DMF (500 ml), insoluble purities were removed by filtration. The DMF was distilled off, and to the residual NMP solution, methanol was added to give gel-like powder. The powder was recovered by filtration and washed with methanol to obtain the required product (2.2 g, 80.5%).

Amino acid analysis [Hydrolysis with 6N HCl]

Arg: $0.96 \times 3$,   Asp: $1.03 \times 2$,   Ser: $0.92 \times 4$   Glu: 1.06,   Gly: $1.01 \times 5$,   Ala: 1.00,   Cys: small peak,   Met: 0.64,   Ile: 0.92,   Leu: 1.03,   Phe: $1.00 \times 2$

Elementary analysis

Found      C 56.30%,   H 6.46%,   N 12.69%

Calculated   C 56.55%,   H 6.50%,   N 12.39%

(for $C_{18}H_{234}O_{40}N_{34}S_6 \cdot 7H_2O$)

(27) Synthesis of α-hANP (5—28)

The protective peptide (483 mg, 10.13 mmole) produced in step (26) was reacted with TFA (5 ml) for 10 minutes with cooling and then for 50 minutes at room temperature, and 3.5N HCl in dioxane (0.1 ml) was added thereto. The solvent was distilled off and to the thus obtained residue ether was added to give a powder. The powder dried overnight over NaOH, and then reacted with HF (7 ml) for 60 minutes at a temperature of −1 to 0°C in the presence of anisole (1.25 ml). The excess HF was distilled off therefrom and the remaining substance was dissolved in about 50% acetic acid and washed three times with ether. The residue was passed through a column ("Dowex 1 × 2", AcO⁻, volume: about 50 ml) to adsorb the required product, and the column was eluted with 5% acetic acid to give a crude powder (reduction product). The powder was dissolved in 1N acetic acid containing urea (13 ml) and the solution thus produced was added dropwise to 1M NH₄OAc/8M urea (pH 7.4, 117 ml) containing $K_3Fe(CN)_6$ (60 mg, 0.18 mmole) with adjustment of the solution to a pH of 7.4 with 10% NH₄OH. After 30 minutes from the completion of the addition, the solution was adjusted to a pH of 4.75 and the resin "IRA-45" (Cl⁻, 10 ml) was added thereto, and then it was passed through a column of the resin "IRA-45" (Cl⁻, 50 ml) to adsorb the product. The column was eluted with 1N acetic acid. The eluant thus obtained was fed to a column of the resin "HP-20" (about 100 ml) and the column was washed. The column was eluted with $CH_3CN/H_2O$/acetic acid (8/1/1) and the eluant thus obtained was concentrated and freeze-dried from 1% acetic acid to give a crude powder (oxidation product, 285 mg). The product (229 mg) was purified with "CM-cellulose" as an eluting agent using 0.05 M (ph 4.7) → 0.5M (pH 4.8) NH₄OAc, and then purified with "HP-20" as eluting agent using 0 → 23% $CH_3CN$/5% acetic acid. The main fraction (49 mg) in the resin "HP-20" was dissolved in TFA (3 ml), aqueous NH₄I (40 mg per 1 ml of water; 20 microlitres, 5.52 micromole) was added thereto with cooling and the mixture was stirred for 10 minutes on a cooling bath. Water and CCl₄ were added thereto with cooling using dry ice as a refrigerant, and the mixture was stirred slowly. When it was moved into a shaker, a gel started to precipitate slowly. Washing with CCl₄ was continued as it was, and the solution containing the gel was fed to a column of the resin "HP-20" (about 20 ml) to desalt it by elution with 5% acetic acid. It was eluted with $CH_3CN/H_2O$/AcOH (8/1/1) and the eluent thus obtained was concentrated and then dissolved in 5% acetic acid. This solution was passed through a column of the resin "Dowex 1 × 2" (AcO, about 25 ml) and eluted with 5% acetic acid, and the element was freeze-dried. The product was purified with the resin "Sephadex LH-20" as an eluting agent using 2N acetic acid to obtain the required product (30 mg).

Amino acid analysis [Hydrolysis with 6N HCl]

NH$_3$: 1.34 × 2,  Arg: 1.03 × 3,  Asp: 1.00 × 2,  Ser: 0.93 × 4,  Glu: 1.02,  Gly: 1.00 × 5,  Ala: 1.02,  Cys: 0.51 × 2,  Met: 0.63,  Ile: 0.94,  Ala: 1.01,  Cys: 1.02 × 2

Elementary analysis

Found        C 43.31%,  H 6.73%,  N 16.49%

Calculated   C 43.33%,  H 6.92%,  N 16.68%

(for C$_{97}$H$_{154}$O$_{32}$N$_{34}$S$_3$·3AcOH.15H$_2$O)

## Example 2
### Synthesis of α-hANP (5—28)

(28) Synthesis of Boc-Cys (4-MeBzl)-Asn-Ser(Bzl)-Phe-Arg(Tos)-Tyr(Cl$_2$Bzl)-OBzl

(1) Boc-Arg(Tos)-Tyr(Cl$_2$Bzl)-OBzl

Boc-Arg(Tos)-OH (2.97 g, 6.93 mmole), H-Tyr (Cl$_2$Bzl)-OBzl·HCl (2.95 g, 6.3 mmole) and HOBt (936 mg, 6.93 mmole) were suspended in a mixture of CH$_2$Cl$_2$ (25 ml) and DMF (10 ml), and WSCI (1.3 ml, 6.93 mmole) was added dropwise thereto with cooling (pH = 4) to allow reaction. In about 40 minutes, it turned into a homogeneous solution, which was stirred overnight. It was confirmed that the amino acid ingredients remained in the solution, by TLC analysis. Then, Boc-Arg(Tos)-OH (270 mg, 0.63 mmole) and WSCI (115 ml, 0.63 mmole) were further added thereto and the mixture was stirred for 3 hours. CH$_2$Cl$_2$ was distilled off, ethyl acetate (150 ml) was added thereto, the solution was washed with 1N HCl, water, 5% aqueous NaHCO$_3$ and water, and dried with Na$_2$SO$_4$. The ethyl acetate was distilled off and the residual substance was washed with ethyl acetate/ether and with n-hexane by decantation to obtain a powder of the required product (5.2 g, 98.1%).

(29) Synthesis of Boc-Phe-Arg(Tos)-Tur(Cl$_2$Bzl)-OBzl

Boc-Arg(Tos)-Tyr(Cl$_2$Bzl)-OBzl (5.1 g, 6.07 mmole) was treated with TFA (15 ml) for 10 minutes with cooling and then for 35 minutes at room temperature, and 6.9N HCl in dioxane (1.1 ml, 7.28 mmole) was added thereto. The solvent was distilled off therefrom and to the residue, ether was added to give a powder. The powder was recovered by filtration and dried under reduced pressure for 3 hours over NaOH. The powder, together with Boc-Phe-OH (1.79 g, 6.37 mmole) and HOBt (902 mg, 6.68 mmole), was dissolved in DMF (20 ml), WSCI (1.22 ml, 6.68 mmole) was added dropwise thereto with cooling (pH = 4.5) and the solution was stirred overnight. Ethyl acetate (150 ml) was added thereto and the mixture washed with 1N HCl, water, 5% NaHCO$_3$ and water, and dried with MgSO$_4$. Ethyl acetate was distilled off and the residual substance was treated with ethyl acetate/ether and with n-hexane to give a powder and thereby the required product (5.56 g, 92.7%) was obtained.

(30) Synthesis of Boc-Ser(Bzl)-Phe-Arg(Tos)-Tyr(ClBzl)-OBzl

Boc-Phe-Arg(Tos)-Tyr(Cl$_2$Bzl)-OBzl (5.46 g, 5.53 mmole) was treated with TFA (15 ml) for 10 minutes with cooling and for 35 minutes at room temperature, and 6.9N HCl/dioxane (0.96 ml, 6.64 mmole) was added thereto. The solvent was distilled off and to the residual substance ether was added to give a powder. The powder was recovered by filtration and dried for 2 hours over NaOH. The powder, together with Boc-Ser (Bzl)-OH (1.8 g, 6.08 mmole) and HOBt (822 mg, 6.08 mmole), was dissolved in DMF (18 ml), WSCI (1.1 ml, 6.08 mmole) was added dropwise thereto with cooling (pH = 4.5) and the solution was stirred overnight. Ethyl acetate (150 ml) was added thereto and the solution was washed with 1N HCl, water, 5% NaHCO$_3$ and water, and dried with Na$_2$SO$_4$. Ethyl acetate was distilled off therefrom and the residue thus obtained was treated with ethyl acetate and ether to give an oily substance, which was allowed to stand overnight to crystallize it. In the same manner as above, it was recrystallized to give the required product (5.47 g, 85.5%).

(31) Synthesis of Boc-Asn-Ser(Bzl)-Phe-Arg(Tos)-Tyr(Cl$_2$Bzl)-OBzl

Boc-Ser(Bzl)-Phe-Arg(Tos)-Tyr(Cl$_2$Bzl)-OBzl (5.4 g, 4.63 mmole) was reacted with TFA (15 ml) for 10 minutes with cooling and then for 40 minutes at room temperature, and 3.5N HCl in dioxane (1.6 ml, 5.56 mmole) was added thereto. The solvent was distilled off therefrom and to the residue, ether was added to give a powder. The powder was recovered by filtration and dried for 5 hours over NaOH. The powder, together with Boc-Asn-OH (1.2 g, 5.09 mmole) and HOBt (6.88 g, 5.09 mmole), was dissolved in DMF (20 ml), and WSCI (0.93 ml, 5.09 mmole) was added dropwise thereto with cooling. The solution was dehydrated by flashing with toluene and treated with chloroform and with methanol/ether to give a powder and thereby give the required product (5.38 g, 90.9%).

Amino acid analysis [Hydrolysis with 6N HCl]

NH$_3$: 1.15,  Arg: 0.97,  Asp: 1.01,  Ser: 0.89,  Tyr: 0.94,  Phe: 1.00

Elementary analysis

Found        C 60.00%,  H 5.74%,  N 9.83%

Calculated   C 60.09%,  H 5.75%,  N 9.85%

(for C$_{64}$H$_{73}$O$_{13}$N$_9$SCl$_2$)

(32) Synthesis of Boc-Cys(4-MeBzl)-Asn-Ser(Bzl)-Phe-Arg(Tos)-Tyr(Cl₂Bzl)-OBzl

Boc-Asn-Ser(Bzl)-Phe-Arg(Tos)-Tyr(Cl₂Bzl)-OBzl (5.3 g, 4.14 mmole) was treated with TFA (20 ml) for 10 minutes with cooling and for 40 minutes at room temperature, and 3.5N HCl in dioxane (1.42 ml, 5 mmole) was added thereto. The solvent was distilled off therefrom and to the residue thus obtained, ether was added to give a powder. The powder was recovered by filtration and dried over NaOH for 2.5 hours. The powder, together with Boc-Cys-(4-MeBzl)-OH (1.48 g, 4.55 mmole) and HOBt (615 mg, 4.55 mmole), was dissolved in DMF (20 ml), then WSCI (0.83 ml, 4.55 mmole) was added dropwise thereto with cooling (pH = 5) and the mixture was stirred overnight. Ethyl acetate (200 ml) was added thereto and the thus obtained solution was washed with water to precipitate a gel. The mixture thus obtained, as it was, was washed with 1N HCl, water, 5% aqueous NaHCO₃ and water, dehydrated by flashing with toluene, and then treated with chloroform and with methanol/ether to give a powder and thereby to give the required product (5.87 g, 95.4%).

Amino acid analysis [Hydrolysis with 6N HCl]

Arg: 0.97, Asp: 1.01, Ser: 0.85, Cys: small peak, Tyr: 0.9, Phe: 1.00

Elementary analysis

Found       C60.55%,   H 5.83%,   N 9.38%

Calculated  C 60.60%,  H 5.83%,   N 9.42%

(for $C_{75}H_{86}O_{14}N_{10}S_2Cl_2$)

(33) Synthesis of protected α-hANP (5—28)

Boc-Ala-Gln-Ser(Bzl)-Gly-Leu-Gly-Cys(4-MeBzl)-Asn-Ser(Bzl)-Phe-Arg(Tos)-Tyr(Cl₂Bzl)-OBzl

Boc-Cys(4-MeBzl)-Asn-Ser(Bzl)-Phe-Arg(Tos)-Tyr(Cl₂Bzl)-OBzl (5.87 g, 3.95 mmole) was reacted with TFA (20 ml) for 10 minutes with cooling and then for 35 minutes at room temperature, and 3.5N HCl in dioxane (1.35 ml, 4.74 mmole) was added thereto. The solvent was distilled off therefrom and to the residue thus obtained ether was added to give a powder. The powder was recovered by filtration and dried for 4.5 hours over NaOH. The powder, together with the carboxylic acid constituent (3 g, 4.15 mmole) of step (9) above and HOBt (587 mg, 4.35 mmoles), was dissolved in DMF (70 ml), and then WSCI (0.8 ml, 4.35 mmole) was added dropwise thereto with cooling (pH = 4.5) to cause a reaction. After 30 minutes, the mixture gelatinized and after 3 hours it turned into a homogeneous solution. It was stirred overnight as it was. It had a negative response in a fluorescamine test (gel precipitated a little).

The above mixture was poured into dilute aqueous NaHCO₃ to give a powder and the powder thus obtained was recovered by filtration, and washed with water, n-hexane and ether, and dried over P₂O₅. The powder was dissolved repeatedly with warm DMF (total volume: 400 ml) to remove impurities. DMF was distilled off therefrom and the residue thus obtained was treated with warm DMF/methanol to give gel like powder and thereby to obtain the required product (7.76 g, 93.9%).

Amino acid analysis [Hydrolysis with 6N HCl]

Arg: 0.97, Asp: 1.00, Ser: 0.90 × 2, Glu: 0.97, Gly: 1.01, Ala: 1.00, Cys: small peak, Leu: 1.03, Tyr: 0.88, Phe: 0.99

Elementary analysis

Found       C58.94%,   H 6.22%,   N 11.25%

Calculated  C 59.18%,  H 6.12%,   N 11.39%

(as $C_{103}H_{127}O_{22}N_{17}S_2Cl_2$)

(34) Synthesis of Boc-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr(Cl₂Bzl)-OBzl

The peptide (5.8 g, 2.77 mmole) as produced above was treated with TFA (20 ml) for 10 minutes with cooling and then for 55 minutes at room temperature, and then 3.5N HCl in dioxane (0.95 ml, 3.32 mmole) was added thereto. The solvent was distilled off therefrom and to the residue thus obtained ether was added to give a powder. The powder was recovered by filtration and dried over NaOH. The powder, together with the carboxylic acid constituent (3.6 g, 2.91 mmole) of step ([18]) above and HOBt (412 mg, 3.05 mmole) was dissolved in a mixture of NMP (40 ml) and DMF (25 ml), and then WSCI (10.56 ml, 3.05 mmole) was added dropwise thereto with cooling to −8°C to cause a reaction. After 2 hours, the mixture gelatinized and after 5.5 hours NMP (15 ml) was further added theeto and the mixture was stirred overnight to give a homogeneous solution having a negative response in a fluorescamine test. The solution was poured into water cooled with ice to give a powder. The powder thus obtained was recovered by filtration, washed with water, n-hexane and ether, and dried ove P₂O₅. The powder was dissolved separately with warm DMF (total volume: 400 ml) and filtered to remove the impurities. DMF was distilled off therefrom, and the residue thus obtained was treated with warm DMF/methanol to give a gel-like powder of the required product (8.71 g, 97.9%).

Amino acid analysis [Hydrolysis (8.71 g, 97.9%]
Arg: 0.96 × 3, Asp: 1.01 × 2, Ser: 0.91 × 2, Glu: 1.04, Gly: 1.02 × 3, Ala: 1.00, Cys: small peak, Met: 0.65, Ile: 0.92, Leu: 1.02, Tyr: 0.96, Phe: 1.00

Elementary analysis
Found        C 56.30%,   H 6.36%,   N 12.69%
Calculated   C 56.32%,   H 6.24%,   N 12.50%
(for $C_{153}H_{197}O_{34}N_{29}S_5Cl_2 \cdot 2.5H_2O$)

(35) Synthesis of Boc-Ser(Bzl)-Ser(Bzl)-Cys(4-MeBzl)-Phe-Gly-Gly-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr-(Cl$_2$Bzl)-OBzl

The peptide produced above (2.4 g, 0.75 mmole) was treated with TFA (10 ml) for 10 minutes with cooling and then for 50 minutes at room temperature, and 3.5N HCl in dioxane (0.26 ml, 0.9 mmole) was added thereto. The solvent was distilled off therefrom and to the residue thus obtained ether was added to give a powder. The powder was recovered by filtration and dried over NaOH. The powder, together with the carboxylic acid constituent (741 m, 0.79 mmole) of step (25) above and HOBt (111 mg, 0.83 mmoles) was dissolved in NMP (35 ml), and then WSCI (150 microlitres 0.83 mmole) was added dropwise thereto with cooling (pH = 5) to cause a reaction. After 2 hours, the mixture gelatinized and after 5 hours NMP (10 ml) was further added thereto. The mixture was stirred overnight and had a negative response in a fluorescamine test. Water was added thereto with cooling and the thus powdered substance was recovered by filtration, washed with water, n-hexane and ether and dried over $P_2O_5$. It was purified by treating with slightly heated DMF/methanol to give a required product (2.74 g, 91.3%).

Amino acid analysis [Hydrolysis with 6N HCl]
Arg: 0.93 × 3, Asp: 1.00 × 2, Ser: 0.90 × 4, Glu: 1.04, Gly: 1.00 × 5, Ala: 1.0, Cys: 0.16 × 2, Met: 0.68, Ile: 0.93, Leu: 0.99, Tyr: 0.99, Phe: 0.97

Elementary analysis
Found        C 58.40%,   H 6.24%,   N 12.14%
Calculated   C 58.56%,   H 6.16%,   N 12.13%
(as $C_{197}H_{247}O_{42}N_{35}S_6Cl_2$)

(36) Synthesis of α-hANP (5—28)

The protected peptide as produced above (525 mg, 0.13 mmole) was reacted to remove its Boc-group in the same manner as the above mentioned method for hANP (5—27). The powder thus obtained was reacted with HF (7.9 ml) in the presence of anisole (1.4 ml) for 60 minutes at a temperature of −2 to −1°C, and excess HF was distilled off. The residue thus obtained was dissolved in about 50% acetic acid, and the solution was washed three times with ether and passed through a column of the resin "Dowex 1 × 2" (AcO, about 30 ml) and then eluted with 10% acetic acid to obtain a crude powder (reduction product). The powder was oxidized in the same manner as the above mentioned method for hANP (5—27) to obtain a crude powder in oxidised form. The powder was purified by a column of CM—C using an eluting agent 0.05M (pH 4.7) → 0.5M (pH 4.8) NH$_4$OAC, and further purified by a column of the resin "HP—20" using as an eluting agent 0 → 27% CH$_3$CN/5% acetic acid and then by a column of the resin "Sephadex LH-20" using as an eluting agent 2N acetic acid to obtain the required product (42 mg).

Amino acid analysis [Hydrolysis with 6N HCl]
NH$_3$: 1.31 × 2, Arg: 1.01 × 3, Asp: 1.00 × 2, Ser: 0.92 × 4, Glu: 0.97, Gly: 1.00 × 5, Ala: 1.00, Cys: 0.85 × 2, Met: 0.66, Ile: 0.92, Leu: 0.97, Tyr: 0.91, Phe: 1.00 × 2

Elementry analysis
Found        C 45.51%,   H 6.62%,   N 16.45%
Calculation  C 45.21%,   H 6.80%,   N 16.78%

Example 3
Synthesis of (Nle$^{12}$α-hANP (1—28)
Synthesis of Boc-Arg(Tos)-Nle-Asp(OBzl)-Arg(Tos)-Ile-Gly-OH

(37) Synthesis of Boc-Nle-Asp(OBzl)-Arg(Tos)-Ile-Gly-OPac

Asp(OBzl)-Arg(Tos)-Ile-Gly-OPac·HCl (4.14 g, 4.8 mmole), Boc-Nle·dicyclohexyl amine (2.18 g, 5.30 mmole) (which had been desalted previously) and HOBt (0.69 g, 5.06 mmole) were dissolved in DMF (10 ml), and WSCI (0.93 ml, 5.06 mmole) was added dropwise thereto with cooling and stirring (pH = 5 to 6). On the next day, it was confirmed that it had a negative response in a fluoroescamine test. Water was poured into the reaction solution and the solution was extracted with ethyl acetate. The ethyl acetate phase was washed with 5% aqueous NaHCO$_3$, water, 1N HCl and water in the order to precipitate a gel-like substance. Ethyl acetate was distilled off therefrom and ether was added thereto. The thus produced gel-like substance was recovered by filtration, washed with ether, and reprecipitated from methanol/ether to give a yield of 4.12 g (83%).

(38) Synthesis of Boc-Arg(Tos)-Nle-Asp(OBzl)-Arg(Tos)-Ile-Gy-OPac

Boc-Nle-Asp(OBzl)-Arg(Tos)-Ile-Gly-OPac (4.04 g, 3.9 mmole) was reacted with CF$_3$CO$_2$H (18 ml) for 10 minutes with cooling and then for 50 minutes at room temperature, and 6.9N HCl in dioxane (0.7 ml, 4.68 mmole) was added thereto, and the excess acid was distilled off therefrom. To the residue thus obtained ether was added and the powder thus obtained was recovered by filtration and dried over NaOH.

The above powder, together with Boc-Arg(Tos) (1.84 g, 4.29 mmole) and HOBt (0.58 g, 4.29 mmole), was dissolved in DMF (8 ml), and then WSCI (0.79 ml, 4.29 mmole) was added dropwise thereto with cooling and stirring (pH = 4 to 5). On the next day, Boc-Arg(Tos) (0.1 g), HOBt (40 mg) and WSCI (40 ml) were further added thereto. After 4 hours it was confirmed that the mixture had a negative response in a fluorescamine test. Water was added thereto and the solid substance thus precipitated was recovered by filtration, washed with water, and then with ether, and reprecipitated from CHCl₃-methanol/ether to give a yield of 4.8 g (91%).

(39) Synthesis of Boc-Arg(Tos)-Nle-Asp(OBzl)-Arg(Tos)-Ile-Gly-OH

Boc-Arg(Tos)-Nle-Asp(OBzl)-Arg(Tos)-Ile-Gly-OPac (3.36 g, 2.5 mmole) was dissolved in acetic acid (25 ml) and powdered zinc (3.3 g) was added thereto. The mixture was stirred for 50 minutes at 45°C. The zinc was removed by filtration and thereafter acetic acid was distilled off therefrom. Water was added thereto, and the solid substance thus precipitated was recovered by filtration, washed with water and ether, and reprecipitated from chloroform-methanol/ether to obtain the required product (2.9 g, 94.5%).

Elementary analysis

Found      C 53.80%,   H 6.78%,   N 13.54%

Calculated   C 54.00%,   H 6.80%,   N 13.50%

(for $C_{56}H_{82}O_{15}N_{12}S_2 \cdot H_2O$)

Amino acid analysis [6N HCl, 110°C for 23 hours and phenol extraction]

Arg: 0.93 × 2,   Asp: 0.98,   Gly: 1.00,   Ile: 0.88,   Nle: 1.01

(40) Synthesis of Boc-Arg(Tos)-Ser(Bzl)-Ser(Bzl)-Cys(4-MeBzl)-Phe-Gly-Gly-OPac

A mixture of Boc-Ser(Bzl)-Ser(Bzl)-Cys(4-MeBzl)-Phe-Gly-Gly-OPac (2.5 g, 2.36 mmole) and TFA (10 ml) was stirred for 50 minutes. TFA was distilled off therefrom, and to the residue thus obtained, 3.5N HCl in dioxane (1 ml, 2.54 mmole) was added, and after sufficient stirring ether was added thereto. The precipitate thus produced was recovered by filtration, dried and dissolved in DMF (10 ml). HOBt (392 mg, 2.9 mmole), Boc-Arg(Tos)-OH (1.24 g, 2.9 mmole), and WSCI (0.53 ml, 2.9 mmole) were added to the above solution with cooling to −15°C. After 16 hours of stirring, water was added to the reaction solution and the substance thus precipitated was recovered by filtration, and recrystallized twice from methanol to give the required product (3.0 g, 92.9%).

(41) Synthesis of Boc-Arg(Tos)-Arg(Tos)-Ser(Bzl)-Ser(Bzl)-Cys(4-MeBzl)-Phe-Gly-Gly-OPac

A mixture of Boc-Arg(Tos)-Ser(Bzl)-Ser(Bzl)-Cys(4-MeBzl)-Phe-Gly-Gly-OPac (2.9 g, 2.1 mmole) and TFA (10 ml) was mixed for 50 minutes. TFA was distilled off therefrom, and to the residue 3.5N HCl in dioxane (0.9 ml, 3.15 mmole) was added, and after sufficient stirring ether was added thereto. The solid substance thus precipitated was recovered by filtration, dried, and then dissolved in DMF (15 ml). HOBt (338 mg, 2.5 mmole), Boc-Arg(Tos)-OH (1.07 g, 2.5 mmole) and WSCI (0.46 ml, 2.5 mmole) were added thereto while cooling to −15°C. After 16 hours of stirring, water was added to the reaction solution and the precipitate thus produced was recovered by filtration, and then treated twice with methanol to obtain the required product (3.1 g, 88.6%).

(42) Synthesis of Boc-Leu-Arg(Tos)-Arg(Tos)-Ser(Bzl)-Ser(Bzl)-Cys(4-MeBzl)-Phe-Gly-Gly-OPac

A mixture of Boc-Arg(Tos)-Arg(Tos)-Ser(Bzl)-Ser(Bzl)-Cys(4-MeBzl)-Phe-Gly-Gly-OPac (3 g, 1.79 mmole) and TFA (10 ml) was stirred for 30 minutes. TFA was distilled off therefrom and to the residue thus obtained, 3.5N HCl in dioxane (0.77 ml, 2.69 mmole) was added and after sufficient stirring ether was added thereto. The precipitate thus produced was recovered by filtration, dried, and dissolved in DMF (15 ml). HOBt (290 mg, 2.15 mmole) and Boc-Leu-OH·H₂O (535 mg, 2.15 mmole), the solution produced by flashing with CHCl₃-toluene Boc-Leu-OH·H₂O (535 mg, 2.15 mmole) and dissolving the thus obtained oily substance in DMF (3 ml), and WSCI (10.39 ml, 2.15 mmole) were added to the solution with cooling to −15°C. After 16 hours of stirring, water was added thereto and the precipitate thus obtained was recovered by filtration and recrystallized twice from methanol to give a yield of 2.5 g (92.2%).

(43) Synthesis of Boc-Ser(Bzl)-Leu-Arg(Tos)-Arg(Tos)-Ser(Bzl)-Ser(Bzl)-Cys-(4-MeBzl)-Phe-Gly-Gly-OPac

A mixture of Boc-Leu-Arg(Tos)-Arg(Tos)-Ser(Bz)-Ser(Bzl)-Cys(4-MeBzl)-Phe-Gly-Gly-OPac (2.9 g, 1.62 mmole) with TFA (10 ml) was stirred for 50 minutes. TFA was distilled off, and to the residue 3.5N HCl in dioxane (0.7 ml, 2.43 mmole) was added and stirred well. The precipitate produced by the addition of ether to the solution was recovered by filtration, dried and dissolved in DMF (15 ml). HOBt (243 mg, 1.8 mmole), Boc-Ser(Bzl)-OH (531 mg, 1.8 mmole) and WSCI (0.33 ml, 1.8 mmole) were added to the above DMF solution with cooling to −15°C. After 16 hours of stirring, water was added to the reaction solution. The substance thus precipitated was recovered by filtration and refluxed twice with methanol to obtain the required product (2.94 g, 91.8%).

14

(44) Synthesis of Boc-Ser(Bzl)-Leu-Arg(Tos)-Arg(Tos)-Ser(Bzl)-Ser(Bzl)-Cys(4-MeBzl)-Phe-Gly-Gly-OH

Boc-Ser(Bzl)-Leu-Arg(Tos)-Arg(Tos)-Ser(Bzl)-Ser(Bzl)-Cys(4-MeBzl)-Phe-Gly-Gly-OPac (2.8 g, 1.42 mmole) was dissolved in acetic acid (80 ml) with heating, powdered zinc (4 g) was added thereto and the mixture was stirred for 1 hour at 45°C. Zinc was removed by filtration, and the acetic acid was distilled off and to the resulting residue water was added. The precipitate thus produced was recovered by filtration, washed with methanol, and then reprecipitated from DMF-MeOH to give the required product in a yield of 2.16 g (82.1%).

Amino acid analysis [Hydrolysis with 6N HCl

Arg: 0.91 × 2, Ser: 0.89 × 3, Gly: 1.00 × 2, Leu: 1.00, Phe: 1.00, 1/2 (Cys)$_2$: small peak

Synthesis of protected (Nle$^{12}$)α-hANP (1—28)

(45) Synthesis of Boc-Arg(Tos)-Nle-Asp(OBzl)-Arg(Tos)-Ile-Gly-Ala-Gln-Se(Bzl)-Gly-Leu-Gly-Cyo(4-MeBzl)-Asn-Ser(Bzl)-Phe-Arg(Tos)-Tyr(OBzl)-OBzl

The peptide produced above in step (33) (1.88 g, 0.9 mmole) was reacted with TFA (10 ml) for 10 minutes with cooling and for 50 minutes at room temperature, and 3.5N HCl in dioxane (0.34 ml, 1.08 mmole) was added thereto. The solvent was distilled off therefrom, and to the residue thus obtained ether was added and the precipitate thus produced was recovered by filtration and dried overnight over NaOH. The precipitate, together with the carboxylic acid constituent of step (39) (1.16 g, 0.95 mmole) and HOBt (134 mg, 0.99 mmole), was dissolved in a mixture of NMP (15 ml) with DMP (12 ml), and then WSCI (180 microlitres 0.99 mmole) was added thereto with cooling (pH = 5.5) to cause a reaction. After 2.5 hours, the mixture gelatinized, and after stirring overnight, it had a negative response in a fluorescamine test. Water was added thereto with cooling and the crystalline substance thus produced was recovered by filtration, washed with water and n-hexane, and dried over P$_2$O$_5$. It was dissolved repeatedly with heated DMF (total volume: 200 ml) and filtered, whereby impurities were removed. DMF was distilled off therefrom and the resulting residue was treated with heated DMF/MeOH to give a gel-like powder of the required product (2.69 g, 93.4%).

Amino acid analysis [6N HCl/PhOH, 108°C, 25 hours]

Arg: 0.91 × 3; Asp: 0.99 × 2, Ser: 0.89 × 2, Glu: 0.97, Gly: 1.00 × 3, Ala: 1.00, Cys: small peak, Ile: 1.02, Leu: 1.08, Nle: 1.05, Tyr: 1.00, Phe: 0.96

Elementary analysis

Found        C 57.54%,   H 6.38%,   N 12.58%
Calculated   C 57.81%,   H 6.27%,   N 12.70%

(46) Synthesis of Boc-Ser(Bzl)-Leu-Arg(Tos)-Arg(Tos)-Ser(Bzl)-Ser(Bzl)-Cys(4-MeBzl)-Phe-Gly-Gly-Arg(Tos)-Nle-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr(OBzl)-OBzl

The peptide produced above in step (45) (1.375 g, 0.43 mmole) was treated with TFA (10 ml) for 10 minutes with cooling and then for 50 minutes at room temperature, and 3.5N HCl in dioxane (0.15 ml, 0.52 mmole) was added thereto. The solvent was distilled off therefrom and to the residue thus obtained ether was added. The powder thus produced was recovered by filtration and dried overnight over NaOH. The powder, together with the carboxylic acid constituent (836 mg, 0.45 mmole) of step (44) above and HOBt (0.47 mmole), was dissolved in NMP (25 ml) (87 ml, 0.47 mmole) was added thereto with cooling (pH = 5) to cause a reaction. After 1.5 hours, the mixture gelatinized, and after stirring overnight as it was, it had a negative response in a fluoroescamine test. Water was added thereto with cooling and the substance thus produced was recovered by filtration, washed with water, n-hexane and ether, and dried over P$_2$O$_5$. It was purified by suspending it in slightly heated DMF and then treating with MeOH to obtain the required product in a yield of 1.97 g (92.9%).

Amino acid analysis [6N HCl/PHOH, 108°C, 25 hours]

Arg: 0.93 × 5, Asp: 1.00 × 2, Ser: 0.89 × 5, Glu: 1.00, Gly: 1.02 × 5, Ala: 1.04, Cys: middle peak, Ile: 0.91, Leu: 1.03 × 2, Nle: 0.95, Tyr: 0.81, Phe: 1.00 × 2

Elementary analysis

Found        C 58.00%,   H 6.41%,   N 12.42%
Calculated   C 58.00%,   H 6.31%,   N 12.68%
(for C$_{240}$H$_{307}$O$_{15}$N$_{45}$S$_7$Cl$_2$·2H$_2$O)

(47) Synthesis of (Nle$^{12}$]α-hANP (5—28)

The protected peptide as produced above (641 mg, 0.13 mmole) was reacted with HF (8.5 ml) for 60 minutes at a temperature of −2 to −1°C, in the presence of anisole (1.7 ml), and the excess HF was distilled off therefrom. After the residue thus obtained had been dissolved in about 50% AcOH, it was washed three times with ether, then passed through a column of the resin "Dowex 1 × 2" (AcO⁻, 50 ml) eluted with 1N AcOH, and then freeze-dried. This product was oxidized in the same manner as the method for the above ANP(5—27) to obtain a crude powder. This product was purified by chromatography with a column of CM-cellulose using as an eluting agent 0.05M (pH 5.0) → 0.7M (pH 5.5) NH$_4$OAc, by chromatography with a column of the resin "HP-20" using as en eluting agent 0 → 27% CH$_3$CN/5% AcOH, and then by chromatography with a column of the resin "Sephadex LH-20" using as an eluting agent 1% AcOH, to obtain the required product in a yield of 83 mg.

Amino acid analysis [Hydrolysis with 6N HCl]

Arg: $1.02 \times 2$, Asp: $1.00 \times 2$, Ser: $0.89 \times 5$, Glu: 0.98, Gly: $0.99 \times 5$, Ala: 1.0, Cys: $0.76 \times 2$, Ile: 0.90, Leu: $1.00 \times 2$, Nle: 0.90, Tyr: 0.86, Phe: $0.98 \times 2$

Elementary analysis

Found        C 45.51%,   H 6.88%,   N 17.67%

Calculated   C 45.37%,   H 6.97%,   N 17.91%

(for $C_{122}H_{194}O_{38}N_{44}S_2 \cdot 4AcOH \cdot 14H_2O$)

## Example 4
### Synthesis of α-hANP (5—25)

Synthesis of Boc-Cys(4-MeBzl)-Asn-Ser(Bzl)-OBzl

(48) Synthesis of Ser(Bzl)-OBzl·TosOH

Boc-Ser(Bzl) (8.0 g, 27.1 mmol) was dissolved in DMF (40 ml), and Bzl-Br (3.3 ml, 27.6 mmole) and $Et_3N$ (3.84 ml, 27.6 mmole) were added thereto with cooling and stirring. Further, $Et_3N$ was added to adjust it to a pH of about 7. On the next day, to the reaction solution water was added, and then the solution extracted with ethyl acetate. The ethyl acetate phase was washed with water, 5% aqueous $NaHCO_3$, 1N HCl and water in the order given and dried over $MgSO_4$. Ethyl acetate was distilled off and the oily substance thus obtained was dissolved in AcOH (20 ml), and then TosOH·$H_2O$ (7.8 g, 40.6 mmole) was added thereto. After 1.5 hours of stirring, AcOH was distilled off, and then to the resulting residue ether was added. The powder thus produced was recovered by filtration, washed sufficiently with ether and then dried over NaOH to give the required product in a yield of 11.15 g (90%).

(49) Synthesis of Boc-Asn-Ser(Bzl)-OBzl

Ser(Bzl)-OBzl·TosOH (11.15 g, 24.4 mmole), Boc-Asn (6.22 g, 26.8 mmole) and HOBt (3.7 g, 26.8 mmole) were dissolved in DMF (30 ml), and WSCI (490 ml, 26.8 mmole) was added dropwise thereto with cooling and stirring. After the completion of the addition, the pH of the solution was about 5. After three hours, it was confirmed that it had a negative response in a fluorescamine test. To the reaction solution, water was added, and the separated oily substance was obtained by extraction with ethyl acetate. The ethyl acetate layer was washed with water, 5% aqueous $NaHCO_3$, water, 1N HCl, and water in the order given, and dried with $MgSO_4$. The ethyl acetate was distilled off therefrom, and the solid residue thus obtained was reprecipitated twice from ethyl acetate/n-hexane to give the required product in a yield of 11.13 g (91%).

(50) Synthesis of Boc-Cys(4-MeBzl)-Asn-Ser(Bzl)-OBzl

Boc-Asn-Ser(Bzl)-OBzl (10.0 g, 20.0 mmole) was reacted with TFA (45 ml, 0.60 mmole) for 10 minutes with cooling and for 50 minutes at room temperature. 3.5N HCl in dioxane (7.0 ml, 24.0 mmole) was added thereto, the excess acid was distilled off therefrom, and to the residue ether was added. The crystalline substance thus precipitated was recovered by filtration, and dried over NaOH. The crystalline substance, Boc-Cys(4-MeBzl) (6.84 g, 21.0 mmole), and HOBt (2.84 g, 21.0 mmole) were dissolved in DMF (30 ml), and WSCI (3.85 ml, 21.0 mmole) was added dropwise thereto while cooling and stirring. The solution was adjusted to a pH of about 4 by the addition of $Et_3N$. On the next day, WSCI (0.37 ml, 2.0 mmole) was further added thereto.

After two hours, it was confirmed that the solution had a negative response in a fluorescamine test. Water was poured into the solution, and the solid thus precipitated was recovered by filtration, washed with water and ether, and then reprecipitated repeatedly from $CHCl_3$-MeOH/AcOEt to give the required product in a yield of 12.6 g (89%).

(51) Synthesis of Boc-Ala-Gln-Ser(Bzl)-Gly-Leu-Gly-Cys(4-MeBzl)-Asn-Ser(Bzl)-OBzl (17—25)

Boc-Cys(4-MeBzl)-Asn-Ser(Bzl)-OBzl (0.885 g, 1.25 mmole) was reacted with TFA (4 ml, 50 mmole) for 10 minutes with cooling and for 50 minutes at room temperature, and 6.9 N HCl in dioxane (0.22 ml, 1.5 mmole) was added thereto. The excess acid was distilled off and to the resulting residue ether was added. The thus produced powdered substance was recovered by filtration and dried over NaOH. The powder, together with Boc-Ala-Gln-Ser(Bzl)-Gly-Leu-Gly-OH (0.95 g, 1.31 mmole) and HOBt (0.19 g, 1.38 mmole), was dissolved in DMF (30 ml), and WSCI (0.252 ml, 1.38 mmole) was added thereto with cooling and stirring (pH about 6). After two hours, it was confirmed that the solution had a negative response in a fluorescamine test. Water was added and the thus precipitated solid was recovered by filtration, washed with water and ether, and reprecipitated from DMF/MeOH to give the required product in a yield of 1.57 g (96%).

Amino acid analysis [6N HCl, 110°C, 22 hours]

$NH_3$: $1.09 \times 2$, Asp: 0.99, Ser: $0.91 \times 2$, Glu: 1.01, Gly: $1.00 \times 2$, Ala: 1.02, Leu: 1.00.

16

(52) Synthesis of Boc-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-OBzl (11—25)

Boc-Ala-Gln-Ser(Bzl)-Gly-Leu-Gly-Cys(4-MeBzl)-Asn-Ser(Bzl)-OBzl (1.47 g, 1.12 mmole) was reacted with TFA (9 ml, 112 mmole) for 10 minutes with cooling and then for 50 minutes at room temperature, and 6.9 N HCl in dioxane (0.25 ml, 1.68 mmole) was added thereto. The excess acid was distilled off therefrom and to the resulting residue ether was added. The powder thus precipitated was recovered by filtration and dried over NaOH. The powder, together with Boc-Arg(Tos)-Met-Asp(OBzl)-Arg(Tos)Ile-Gly-OH (1.47 g, 1.18 mmole) and HOBt (0.17 g, 1.23 mmole), was dissolved in a mixture of DMF (15 ml), and NMF (5 ml), and WSCI (0.226 ml, 1.23 mmole) was added thereto with cooling and stirring (pH about 5). On the next day, it was confirmed that the solution had a negative response in a fluorescamine test. To the pudding-like reaction product, water was added and the solid thus precipitated was recovered by filtration, washed with water and ether, and then reprecipitated from DMF/MeOH to give the required product in a yield of 2.57 g (94%).

Amino acid analysis [6N HCl, 110°C, 22 hours]

NH$_3$: 1.13 × 2, Arg: 0.89, Asp: 1.00 × 2, Ser: 0.90 × 2, Glu: 1.04, Gly: 1.01 × 3 Ala: 1.01, Met: 0.39, Ile: 0.93, Leu: 1.00.

(53) Synthesis of Boc-Ser(Bzl)-Ser(Bzl)-Cys(4-MeBzl)-Phe-Gly-Gly-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-OBzl (5—25)

Boc-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-OBzl (2.43 g, 1.0 mmole) was reacted with TFA (12 ml) for 10 minutes with cooling and for 50 minutes at room temperature, and 6.9 N HCl in dioxane (0.22 ml, 1.5 mmole) was added thereto. The excess acid was distilled off therefrom. Ether was added to the residue to give a powder, and the powder was dried over NaOH. The powder, together with Boc-Ser(Bzl)-Ser(Bzl)-Cys(4-MeBzl)-Phe-Gly-Gly-OH (0.99 g, 1.05 mmole) and HOBt (0.15 g, 1.10 mmole), was dissolved in a mixture of DMF (5 ml), and NMP (20 ml) and WSCI (0.201 ml, 1.10 mmole) was added thereto with cooling and stirring (pH 5 to 6). After 4 hours, it was confirmed that the solution had a negative response in a fluorescamine test. To the pudding-like reaction product, water was added and the solid thus precipitated was recovered by filtration, washed with water, n-hexane and ether in the order given, and then reprecipitated from DMF/MeOH to give the required product in a yield of 3.0 g (92%).

Amino acid analysis [6N HCl, 110°C, 22 hours]

NH$_3$: 1.26 × 2, Arg: 0.93 × 2, Asp: 1.01 × 2, Ser: 0.92 × 4, Glu: 1.03, Gly: 1.00 × 5 Ala: 1.00, 1/2Ca (Cys)$_2$: small peak, Met: small peak, Ile: 0.98, Leu: 1.08, Phe: 0.96.

(54) Synthesis of α-hANP (5—25)

The protected peptide produced above (1.0 g, 0.307 mmole) was treated with TFA (5 ml) for 10 minutes at 0°C and for 50 minutes at room temperature, and 6.9N HCl in dioxane (0.1 ml, 0.76 mmole) was added thereto. The excess acid was distilled off and to the residue thus produced ether was added to give a powder. The powder was recovered and dried over NaOH. The powder was treated with HF (about 9 ml) in the presence of anisole (1.5 ml, 13.8 mmole) for 1 hour at 0°C. HF was distilled off therefrom, and the oily residue was dissolved in 2N AcOH, and washed with ether. The aqueous layer was passed through a column of the resin "Dowex 1×2" (AcO$^-$, 100 ml) and eluted with 1N HCl. The fractions with a positive response in a fluorescamine test were collected, combined together and freeze-dried.

The freeze-dried substance was dissolved in 1N AcOH/urea and the solution was added dropwise to 1M AcONH$_4$ (pH 7.4)/8M urea (270 ml) containing K$_3$Fe(CN)$_6$ (0.142 g, 0.43 mmole) over a period of 10 minutes. During this addition, the solution was kept to a pH of 7.4 by adding 10% aqueous ammonia, dropwise.

Thereafter, the solution, was stirred for 10 minutes, and its pH was adjusted to 5 by adding acetic acid. The resin "IRA-45" (Cl$^-$, about 20 ml) was added thereto, and the mixture was stirred slowly for 10 minutes. This solution was fed to a column of the resin "IRA-45" (Cl$^-$, 100 ml) and then to a column of the resin "HP-20" (fine, 150 ml), and the two columns were washed with 1N AcOH (600 ml). The column of the resin "HP-20" was eluted with CH$_3$CN/AcOH/H$_2$ (8:1:1) (500 ml). The solution thus eluted was concentrated, and the oily residue thus obtained was freeze-dried with 1N AcOH.

The freeze-dried substance was dissolved in TFA (30 ml), and NH$_4$I (95 mg, 0.61 mmole)/H$_2$O (1 ml) was added thereto with cooling and stirring. After 10 minutes, water (400 ml) was added to the solution, and the solution thus obtained was washed with CCl$_4$. To the aqueous layer, ascorbic acid (55 mg) was added, and then the solution thus obtained was passed through a column of the resin "HP-20" (fine, 100 ml). The column was washed with 1N AcOH (500 ml), and then eluted with CH$_3$CN/AcOH/H$_2$O (8:1:1) (400 ml). The eluents thus obtained were combined together and concentrated. The resulting oily residue was dissolved in 1N AcOH and this solution was passed through a column of the resin "Dowex 1×2" (AcO-, 100 ml). The column was eluted with 1N AcOH. The fractions whose solution and a positive response in a fluorescamine test, were collected, combined together, and freeze-dried.

Next, the product was purified by linear gradient elution in a column of CM-cellulose (2.65 cm diameter × 40 cm; 0.03M AcONH$_4$ (pH 4.8); 900 ml → 0.3M AcONH$_4$ (pH 4.8) (900 ml).

Further, the product was eluted by linear gradient elution) in a column of the resin "HP-20" (1.9 cm diameter × 50 cm; 0% CH$_3$CN/1% AcOH (600 ml) 20% CH$_3$CN/1% ACOH (600 ml). The mixture of the required fractions was concentrated and freeze-dried with 1N AcOH. The freeze-dried substance was purified by further elution with a column of the resin "LH-20" (2.13 cm diameter × 46 cm) using as an eluting agent 1N HCOH.

By use of the purification system described above, the required product was obtained, in purified form, in a yield of 75 mg (10.3%).

Elementary analysis
Found:         C 42.57%,   H 6.54%,   N 16.81%
Calculated:   C 42.43%,   H 6.83%,   N 17.09%
(for $C_{82}H_{133}N_{29}O_{30}S_3 \cdot AcOH \cdot 12H_2$)

Amino acid analysis [6N HCl, 110°C, 22 hours]
$NH_3$: 2.20 × 2, Arg: 0.94 × 2, Asp: 1.00 × 2, Ser: 0.74 × 4, Glu: 1.0, Gly: 0.99 × 5, Ala: 1.02, 1/2 $(Cys)_2$: 0.40 × 2, Met: small peak, Ile: 0.91, Leu: 0.96, Phe: 0.96.

Example 5 — Synthesis of α-hANP (7—28)

(55) Synthesis of Boc-Cys(4-MeBzl)-Phe-Gly-Gly-OH

Boc-Cys(4-MeBzl)-Phe-Gly-Gly-OPac (3.2 g, 4.5 mmole) was dissolved in acetic acid (50 ml) and powdered zinc (8 g) was added thereto. The mixture was stirred for 50 minutes at 45°C. The zinc was removed by filtration, and then acetic acid was distilled off therefrom. To the resulting residue water was added, and the precipitate thus produced was recovered by filtration, dried, and reprecipitated from methanol/-ether to give the required product in a yield of 2.27 g (87.3%).

(56) Synthesis of protected α-hANP (7—28)
Synthesis of Boc-Cys(4-MeBzl)-Phe-Gly-Gly-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(OBzl)-Phe-Arg(Tos)-Tyr($Cl_2$Bzl)-OBzl

Boc-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr($Cl_2$Bzl)-OBzl (2.09 g, 0.65 mmole) was mixed with TFA (15 ml), and the mixture was stirred for 50 minutes. TFA was distilled off and to the residue 3.5N HCl in dioxane (0.28 ml, 0.98 mmole), was added and stirred well. Ether was added thereto. The precipitate thus produced was recovered by filtration, dried, and dissolved in NMP (25 ml), and HOBt (97 mg, 0.72 mmole), Boc-Cys(4-MeBzl)Phe-Gly-Gly-OH (422 mg, 0.72 mmole) and WSCI (140 microlitres, 0.72 mmole) were added to the solution with cooling to −15°C. After 16 hours of stirring, to the gelatinized reaction solution water was added, the substance thus precipitated was recovered by filtration, washed with MeOH to give the required product in a yield of 2.2 g (91.6%).

(57) Synthesis of Cys-Phe-Gly-Gly-Arg-Met-Asp-Arg-Ile-Gly-Ala-Gln-Ser-Gly-Leu-Gly-Cys-Asn-Ser-Phe-Arg-Tyr
[α-hANP (7—28)]

To Boc-Cys(4-MeBzl)-Phe-Gly-Gly-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr($Cl_2$Bzl)-OBzl (1 g, 0.27 mmole), TFA (5 ml) was added and the mixture was stirred for 50 minutes. TFA was distilled off and to the residue thus obtained ether was added. The substance thus precipitated was recovered by filtration, dried and then treated with anisole (2 ml) and HF (20 ml) for 60 minutes at 0°C. HF was distilled off and to the residue ether was added. The substance thus precipitated was washed with ether, dissolved in 20% acetic acid, and then passed through a column of the resin "Dowex 1 × 2" ($ACO^-$). The column was eluted with 1N AcOH. The eluents were collected, combined together and freeze-dried. All of the thus obtained powder was dissolved in 1N ACOH (27 ml) and the solution was added dropwise to a mixture of 1M $AcONH_4$/urea solution (243 ml) and $K_3Fe(CN)_6$ (125 mg). The solution was adjusted to pH 4 with 10% $NH_4OH$. In 30 minutes, the dropping was ended, and the solution was adjusted to pH 4.75 with conc. ACOH, mixed with the resin "IRA-45" ($Cl^-$, 20 ml) and then stirred slowly. Next, the solution was passed through a column of the resin "IRA-45" ($Cl^-$, 100 ml), and washed with 1N ACOH. The washing liquid was treated with the resin "HP-20" to adsorb the product and then the resin was washed with 1% AcOH. The resin was eluted with $CH_3CN$/AcOH/water (8:1:1) and the eluent thus obtained was freeze-dried. The freeze-dried powder was purified by gradient elution with CM-cellulose-column using as an eluting agent 0.05M → 0.5M $NH_4OAc$. The main fractions (fractions 60 to 67) were collected and freeze-dried. Next, the product was purified with a column of the resin "HP-20" (5% $CH_3CN$ → 25% $CH_3CN$). The main fractions (fractions 70 to 82) were collected and freeze-dried. In the end, the product was again purified with a column of the resin "LH-20" and 2M AcOH to give the required purified product in a yield of 78 mg.

Amino acid analysis [Hydrolysis with 6N HCl]
Arg: 0.98 × 3, Asp: 0.99 × 2, Ser: 0.86 × 2 Glu: 1.00, Gly: 0.98 × 5, Ala: 1.0, 1/2 $(Cys)_2$: 0.84 × 2, Met: 0.71, Ile: 0.93, Leu: 1.00, Tyr: 0.90, Phe: 0.99 ×
HPLC [Column: Nucleosil $5C_{18}$; 0.1% TFA (1% 60% gradient)]
Single peak (26.0 minutes).

## Example 6
### Synthesis of (Asu$^{7,23}$)α-hANP (7—23)

(58) Synthesis of Boc-Ala-Gln-Ser(Bzl)-Gln-Leu-Gly-Asu(OPac)-OBzl

Boc-Ala-Gln-Ser(Bzl)-Gly-Leu-Gly-OH (1.23 g, 1.7 mmole), p-ToSOH·Asu(OPac)-OBzl (1.03 g, 1.8 mmole) and HOBt (0.26 g, 1.9 mmole) were dissolved in DMF (10 ml) and WSCI (0.35 ml, 1.9 mmole) was added thereto at −5°C. The solution was stirred for 1 hour at −5°C and then overnight at room temperature. The solution was poured into cooled water (100 ml), and the solid thus precipitated was recovered by filtration, washed with water and with ether, and then reprecipitated from chloroform-methanol/ether to give the required product in a yield of 1.65 g (88%).

Amino acid analysis [6N HCl, — phenol/110°C, 22 hours]

NH$_3$: 1.08, Ser: 0.92, Glu: 0.98, Gly: 1.03 × 2, Ala: 1.00, Asu: 1.03, Leu: 1.

HPLC

Retention time in HPLC of the product: 4.5 minutes; Resin: Nucleosil 5C$_{18}$, (hereinafter, the same resin is used for HPLC unless otherwise stated).

Eluting agent: MeOH/H$_2$O/TFA (80/20/0.1).

Eluting method: isocratic

(59) Synthesis of Boc-Arg(Tos)-Met-Asp(OBzl)-Arg(Tos)-Ile-Gly-Ala-Gln-Ser(Bzl)-Gly-Leu-Gly-Asu(OPac)-OBzl

To Boc-Ala-Gln-Ser(Bzl)-Gly-Leu-Gly-Asu(OPac)-OBzl (1.5 g, 1.36 mmole), TFA (10 ml) was added, and the mixture was stirred for 10 minutes at −5°C and then for 30 minutes at room temperature. The excess TFA as distilled off under reduced pressure and to the resulting residue 3.5N HCl in dioxane (0.5 ml) was added to change the free amino group to hydrochloride. To the solution, ether was added and the solid thus precipitated was recovered by filtration and dried under reduced pressure for 5 hours over NaOH. The solid obtained was dissolved in DMF (15 ml), and Boc-Arg(Tos)-Met-Asp(OBzl)-Arg(Tos)-Ile-Gly-OH (1.78 g, 1.43 mmole) and HOBt (0.20 g, 1.5 mmole) were added thereto, and further WSCI (0.28 ml, 1.5 mmole) was added thereto at −5°C. The mixture was stirred for 1 hour at −5°C and then overnight at room temperature. The reaction solution was poured into cooled water (200 ml). The solid thus precipitated was recovered by filtration, washed with water and with ether, and reprecipitated from DMF/MeOH to give the required product in a yield of 2.6 g (86%).

Amino acid analysis [6N HCl — phenol/110°C, 22 hours]

NH$_3$: 1.13, Arg: 0.96 × 2, Asp: 1.00, Ser: 0.91, Glu: 1.00, Gly: 1.02 × 3, Ala: 0.98, Met: 0.62, Asu: 1.02, Ile: 1.01, Leu: 1.08.

HPLC

Retention time: 12 minutes 45 seconds

Eluting agent: MeOH/H$_2$O/TFA (80/20/0.1)

Eluting method: isocratic

(60) Synthesis of Boc-Phe-Gly-Gly-OH

Boc-Phe-Gly-Gly-OPac (2.5 g, 5 mmole) was dissolved in acetic acid (50 ml), and powdered zinc was added thereto. The mixture was stirred for 40 minutes at 45°C. The zinc was removed by filtration, and the solution was concentrated by distilling off acetic acid. To the resulting residue, water was added, and the oily substance thus precipitated was obtained by extraction with ethyl acetate. The ethyl acetate layer was washed with 1N HCl and water, and dried with Na$_2$SO$_4$. Ethyl acetate was distilled off threrefrom. The resulting residue was recrystallized from ethyl acetate/n-hexane to give the required product in a yield of 1.75 g (92.1%).

(61) Synthesis of Boc-Phe-Gly-Gly-Arg(Tos)-Met-Asp-(OBzl)-Arg(Tos)-Ile-Gly-Ala-Gln-Ser(Bzl)-Gly-Leu-Gly-Asu(OPac)-OBzl

To Boc-Arg(Tos)-Met-Asp(OBzl)-Arg(Tos)-Ile-Gly-Ala-Gln-Ser(Bzl)-Gly-Leu-Gly-Asu(OPac)-OBzl (2.2 g, 1 mmole), TFA (10 ml) was added and the mixture was stirred for 20 minutes at −5°C and for 30 minutes at room temperature. The excess TFA was distilled off under reduced pressure, and to the residue 2.5N HCl in dioxane (0.4 ml) was added to change the free amino group to the hydrochloride. Ether was added thereto, and the thus precipitated solid was recovered by filtration, dried under reduced pressure for 5 hours over NaOH, and dissolved in DMF (30 ml). Boc-Phe-Gly-Gly-O (0.42 g, 1.1 mmole) and HOBt (0.15 g, 1.1 mmole) were added thereto and further WSCI (0.20 ml, 1.1 mmole) was added thereto at −5°C. The mixture was stirred for 1 hour at −5°C and overnight at room temperature. A solution obtained by dissolving Boc-Phe-Gly-Gly-OH (38 mg, 0.1 mmole) and HOBt (14 mg, 0.1 mmole) in DMF (5 ml) was added to the reaction solution as produced above, and WSCI (19 microlitres, 0.1 mmole) was further added thereto at −5°C. The solution thus obtained was stirred for 1 hour at −50°C and for 3 hours at room temperature, and then poured into cooled 2.5% aqueous sodium bicarbonate (300 ml). The precipitate thus produced. was recovered by filtration, washed with water and ether, refluxed with MeOH (200 ml); cooled naturally, and recovered by filtration to give the required product in a yield of 2.3 g (92%).

Amino acid analysis [6N HCl-phenol/110°C, 22 hours]

NH$_3$: 1.07, Arg( 0.93 × 2, Asp: 1.00, Ser: 0.87 Glu: 0.99, Gly: 1.03 × 5, Ala: 1.04, Met: 0.51, Asu: 0.99, Ile: 0.97, Leu: 1.04, Phe: 0.94.

HPLC

Retention time: 9 minutes 40 seconds

Eluting agent: MeOH/H$_2$O/TFA (82.1/27.5/0.1)

Eluting method: isocratic

(62) Synthesis of Boc-Phe-Gly-Gly-Arg(Tos)-Met-Asp-(OBzl)-Arg(Tos)-Ile-Gly-Ala-Gln-Ser(Bzl)-Gly-Leu-Gly-Asu(OH)-OBzl

Boc-Phe-Gly-Gly-Arg(Tos)-Met-Asp(OBzl)-Arg(Tos)-Ile-Gly-Ala-Gln-Ser(Bzl)-Gly-Leu-Gly-Asu(OPac)-OBzl (1.7 g, 0.7 mmole) was dissolved in AcOH/TFE (80 ml/20 ml), and powdered zinc (2.3 g, 35 mmole) was added thereto. The mixture was stirred for 50 minutes at 49°C. In the solution unreacted substance (Pac-derivative) was detected, and so powdered zinc (2.5 g, 38 mmole) was added thereto, and the solution was stirred for 20 minutes at 49°C. Further, powdered zinc (1 g, 15 mmole) was added thereto, and the solution was stirred for 10 minutes at 49°C. The excess zinc was removed by filtration, and the resulting filtrate was concentrated under reduced pressure. To the residue water was added, and the solid thus precipitated was recovered by filtration, washed with water and ether, refluxed with methanol (100 ml) and cooled naturally to give the required product.

Amino acid analysis [6N HCl-phenol/110°C, 22 hours]

NH$_3$: 1.10 × 2, Arg: 0.95 × 2, Asp: 1.00, Ser: 0.85, Glu: 0.99, Gly: 1.02 ×: Ala: 1.05, Met: 0.71, Asu: 0.99, Ile: 0.99, Leu: 1.07, Phe: 0.98 HPLC

Retention time: 5 minutes 30 seconds

Eluting agent: MeOH/H$_2$O/TFA (82.5/17.5/0.1)

Eluting method: isocratic

A compound obtained by changing Met to Met(O) is much involved in the product (about 30%). The retention time of this product in HPLC, under the same conditions as above, was 4 minutes.

(63) Synthesis of Phe-Gly-Gly-Arg(Tos)-Met-Asp(OBzl)-Arg(Tos)-Ile-Gly-Ala-Gln-Ser(Bzl)-Gly-Leu-Gly-Asu-Obzl

To Boc-Phe-Gly-Gly-Arg(Tos)-Met-Asp(OBzl)-Arg(Tos)-Ile-Gly-Ala-Gln-Ser(Bzl)-Gly-Leu-Gly-Asu(OH)-OBzl (0.67 g, 0.28 mmole), TFA (5 ml) was added and the mixture was stirred for 10 minutes at −5°C and for 50 minutes at room temperature. The excess TFA was distilled off under reduced pressure, and to the residue thus obtained ether was added. The solid thus precipitated was recovered by filtration, dried under reduced pressure for 2 hours over NaOH, and dissolved in DMF (10 ml). Et$_3$N (40 microlitres, 0.28 mmole) was added to the above solution at −5°C, the solution was neutrallized, and further water was added thereto. The solid thus precipitated was recovered by filtration, washed with water and ether, and dried under reduced pressure for 2 days over P$_2$O$_5$. The Boc-removed derivative thus obtained was dissolved in DMF (40 ml), HOBt (57 mg, 0.42 mmole) was added thereto and further WSCl/HCl (81 mg, 0.42 mmole) was added thereto at −5°C. The solution was stirred for 1 hour at −5°C and for 4 hours at room temperature, and HOBt (57 mg, 0.42 mmole) and WSCl·HCl (81 mg, 0.42 mmole) were added thereto at −5°C, and the solution was stirred for 1 hour at −5°C and overnight at room temperature. DMF was distilled off under reduced pressure, and to the residue water was added. The solid thus precipitated was recovered by filtration, washed with water and ether, and reprecipitated from MeOH/AcOEt-ether to give the required product in a yield of 430 mg (68%).

HPLC

Retention time: 19 minutes 50 seconds

Eluting agent: MeOH/H$_2$O/TFA (70/30/0.1) (Called "A")

MeOH/H$_2$O/TFA (95/5/0.1) (Called "B")

Eluting method:

After linear gradient solution with A and B for 15 minutes, the product was eluted with B for 15 minutes.

Under the same conditions as above, the retention time of the Met(O) derivative was 17 minutes 50 seconds, and of the de-Boc derivative was 13 minutes, 40 seconds.

(64) Synthesis of Phe-Gly-Gly-Arg-Asp-Met-Arg-Ile-Gly-Ala-Gln-Ser-Gly-Leu-Gly-Asu-OH

A mixture of

Phe-Gly-Gly-Arg(Tos)-Asp(OBzl)-Met-Arg(Tos)-Ile-Gly-Ala-Gln-Ser-Bzl-Gly-Leu-Gly-Asu-OBzl

(338 mg, 0.15 mmole) and HF (5 ml) was stirred in the presence of anisole (0.3 ml) and methionine (34 mg) for 1 hour on a cooling bath. The excess was distilled off under reduced pressure, and the resulting residue was dissolved in 50% AcOH (5 ml) and then passed through a column of the resin "Dowex 1×2"

(AcO⁻, 50 ml). The eluent thus obtained was concentrated under reduced pressure, and the residue was dissolved in 2M AcOH, and freze-dried to give a crude product. The crude product was purified by chromatography with CM-Cellulose [Column size: 1.75 × 29 cm; eluting solvent: 0.01M AcONH₄ (pH 4.8) (called "A-buffer"); 0.4M AcONH₄ (pH 4.8) (called "B-buffer"); eluting method: linear gradient elution with A-buffer (300 ml) and B-buffer (300 ml)] to give the product in a yield of 90 mg.

The product as partially purified above (40 mg) was purified as follows. First, it was dissolved in TFA (2 ml), and 4% aqueous NH₄I (110 microlitres, 30 micromole) was added thereto. The soliution was stirred for 10 minutes. Second, a large excess of water was added to the reaction solution, and the solution was washed with CCl₄. The separated aqueous layer treated with the resin "Dia-ion HP-20" to adsorb the product. The resin was washed with water and treated with CH₃CN/10% AcOH for desorption. The eluent thus obtained was concentrated under reduced pressure, and the resulting residue was dissolved in 2M AcOH and then passed through a column of the resin "Dowex 1 × 2" (ACO⁻). The eluent thus obtained was freeze-dried. Further, the product was purified by column chromatography using the resin "Dia-ion HP-20" [Column size: 1.75 × 29 cm; eluting solvent: 10% AcOH (Called "A" solution); CH₃CN/10% AcOH (25/75) (called "B solution"); eluting method: linear gradient elution with A solution (300 ml) and B solution (300 ml)].

The fractions containing the required product were collected, combined together and concentrated. The resulting residue was dissolved in 2M ACOH, and freeze-dried. Further, the freeze-dried product was purified by column chromatography using the resin "Sephadex LH-20" [Column size: 1.75 × 29 cm; Eluting solvent: 2M ACOH] to give the required product in a yield of 21 mg and having a specific rotation [a] 30 of −32.0 (C: 0.52M, AcOH).

Amino acid analysis [6N HCl/110°C, 22 hours]
NH₃: 1.33 × 2, Arg: 0.95 × 22, Asp: 0.98, Ser: 0.92, Glu: 0.98, Gly: 1.00 × 5, Ala: 1.00, Met: 0.43, Asu: 0.98, Ile: 0.97, Leu: 1.02, Phe: 0.95.

HPLC
Retention time: 26 minutes
Eluting agent: CH₃CN/H₂O/TFA (1/99/0.1) (called "A")
CH₃CN/H₂O/TFA (60/40/0.1) (called "B")
Eluting method: linear gradient elution for 256 minutes with A and B, and then for 10 minutes with B.

Synthesis of (Nle¹²)-α-hANP (7—28) (see also Example 11 below)

(65) Synthesis Of Protected (Nle¹²)-α-hANP (7—28)
To Boc-Arg(Tos)-Nle-Asp(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr(Cl₂Bzl)-OBzl (650 mg, 0.20 mmole), TFA (5 ml) was added and the mixture was stirred for 50 minutes. TFA was distilled off therefrom, and to the residue 3.5N HCl in dioxane (100 microlitres, 0.35 mmole) was added and mixed well, and ether was added thereto. The substance thus precipitated was recovered by filtration, dried and dissolved in N-methylpyrolidone (15 ml). HOBt (36 mg, 0.26 mmole), Boc-Cys(4-MeBzl)-Phe-Gly-Gly-OH (153 mg, 0.26 mmole) and WSCI (50 microlitres, 0.27 mmole) were added to the above solution with cooling to −15°C. After 16 hours of stirring, to the reaction solution thus gelatinized, water was added. The precipitate thus obtained was recovered by filtration, and washed twice with MeOH with heating to give the required product in a yield of 700 mg.

Amino acid analysis [Hydrolysis with 6N HCl, 110°C, 22 hours]
Arg: 0.99 × 3, Asp: 1.00 × 2, Ser: 0.90 × 2, Glu: 1.00, Gly: 0.98 × 5, Ala: 1.07, 1/2 (Cys)₂: middle peak, Nle: 0.93 g, Ile: 0.93, Leu: 1.00 Tyr: 0.92, Phe: 0.98 × 2.

(66) Synthesis of Cys-Phe-Gly-Gly-Arg-Nle-Asp-Arg-Ile-Gly-Ala-Gln-Ser-Gly-Leu-Gly-Cys-Asn-Ser-Phe-Arg-Tyr [(Nle¹²)-α-hAN: (7—28)]

Protected (Nle¹²)-α-hANP (7—28) (500 mg, 0.136 mmole) was treated with HF (10 ml) in the presence of anisole (1 ml) at 0°C for 60 minutes. HF was distilled off therefrom, and to the residue ether was added. The substance thus precipitated was washed with ether, and dissolved in 20% AcOH. The solution was passed through a column of the resin "Dowex 1 × 2" (ACO⁻), and then eluted with 1N AcOH. The eluents thus obtained were collected, combined together and freeze-dried. All of the powder thus obtained was dissolved in 1N AcOH (14 ml), and the solution was added dropwise to a solution produced by mixing 1M AcONH₄/urea solution (126 ml) with K₃Fe(CN)₆ (63 mg). The solution was adjusted to pH 7.4 with 10% NH₄OH. In 30 minutes, the addition was complete and the solution was adjusted to pH 4.75 with conc. AcOH. Then the resin "IRA-45" (Cl⁻) (15 ml) was added thereto, and the mixture was slowly stirred. Next, the solution was passed through a column of the resin "IRA-45" (Cl⁻) (70 ml) and the column was washed with 1N AcOH. The washing liquid was treated with the resin "HP-20" for adsorption and the resin was washed with 1% AcOH. The resin was eluted with a mixture of CH₃CN/AcOH/H₂O (8/1/1), and the eluent thus obtained was concentrated and freeze-dried. The crude freeze-dried powder was purified by CM—C Cellulose Column chromatography using linear gradient elution and using as an eluting solvent 0.05M (pH 5) → 0.5M NH₄OAc. The main fractions (fractions 50 to 58) were collected, combined together and freeze-dried. Next, the product was further purified by chromatography using gradient elution and using the resin "HP-20" and as an eluting solvent 5% CH₃CN → 25% CH₃CN, and the main fractions (fractions 68

to 81) were collected, combined together, and freeze-dried. Lastly, the product was further purified with the resin "LH-20" and 2M AcOH to give the required product in a yield of 74 mg.

Amino acid analysis [Hydrolysis with 6N HCl]

Arg: 0.99 × 3, Asp: 1.00 × 2, Ser: 0.93 × 2, Glu: 1.00, Gly: 1.00 × 5, Ala: 1.05, 1/2 (Cys)$_2$: 0.86 × 2, Nle: 0.94, Il: 0.94, Leu: 1.00, Tyr: 0.95, Phe: 0.99 × 2.

## Example 7
### Synthesis of [Met(0)$^{12}$]-α-hANP (1—28)

(67) Synthesis of Boc-Ser(Bzl)-Leu-Arg(Tos)-Arg(Tos)-Ser(Bzl)-Ser-Cys(4-MeBzl)-Phe-Gly-Gly-Arg(Tos)-Met-Asp-(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr(Cl$_2$Bzl)-OBzl

A mixture of Boc-Arg(Tos)-Met-Asp-(OBzl)-Arg(Tos)-Ile-Gly-Ala-Gln-Ser(Bzl)-Gly-Leu-Gly-Cys(4-MeBzl)-Asn-Ser(Bzl)-Phe-Arg(Tos)-Tyr(Cl$_2$Bzl)-OBzl (2.02 g, 0.63 g mmole) and TFA (20 ml) was stirred for 50 minutes. TFA was distilled off, and to the resulting residue, 3.5N HCl in dioxane (0.27 ml) was added. The solution was stirred well, and ether was added thereto. The thus precipitated substance recovered by filtration, dried, and dissolved in N-methyl pyrolidone (30 ml). Then, HOBt (89 mg, 0.66 mmole), Boc-Ser(Bzl)-Leu(Tos)-Arg(Tos)-Ser(Bzl)-Ser(Bzl)-Cys(4-MeBzl)-Phe-Gly-Gly-OH (1.22 g, 0.66 mmole) and WSCl (120 microlitres, 0.66 mmole) were added thereto with cooling to −15°C. After 16 hours of stirring, to the thus gelatinized reaction solution, water was added. The thus precipitated substance was recovered by filtration, washed with methanol, then refluxed with methanol to give the required product in a yield of 3.0 g (96.5%).

Amino acid analysis [Hydrolysis with 6N HCl]

Arg: 0.98 × 5, Asp: 1.00 × 2, Ser: 0.90 × 5, Glu: 1.07, Gly: 1.02 × 5, Ala: 1.0 1/2 (Cys)$_2$: 0.19, Met: 0.60, Ile: 0.89, Leu: 1.08 × 2, Try: 0.97, Phe: 1.02 × 2.

(68) Synthesis of [Met(O)$^{12}$]-α-hANP (1—28)

To Boc-Ser(Bzl)-Leu-Arg(Tos)-Arg(Tos)-Ser(Bzl)-Ser(Bzl)-Cys(4-MeBzl)-Phe-Gly-Gly-Arg(Tos)-Met-Asp(OBzl)-Arg(Tos)-Ile-Gly-Ala-Gln-Ser(Bzl)-Gly-Leu-Gly-Cys(4-MeBzl)-Asn-Ser-(Bzl)-Phe-Arg(Tos)-Tyr(Cl$_2$Bzl)-OBzl (1 g, 0.2 mmole), TFA (5 ml) was added. The solution was stirred for 50 minutes. TFA was distilled off, and ether was added to the resulting residue. The precipitate thus obtained was recovered by filtration, dried and reacted with HF (30 ml) in the presence of anisole (1.5 ml) for 60 minutes at 0°C. HF was distilled off therefrom. The residue thus obtained was washed sufficiently with ether, and dissolved in 2N AcOH. The solution was passed through a column of the resin "Dowex 1 × 2" (AcO⁻), and was eluted with 1N AcOH. The eluent thus obtained was concentrated and freeze-dried. The freeze-dried powder was dissolved in 1M acetic acid (920 ml), and this solution was added dropwise to a solution produced by mixing 1M NH$_4$OAc urea solution (180 ml) with K$_3$Fe(CN)$_6$ (93 mg). The solution was maintained at pH 7.4 by the addition of 10% NH$_4$OH. In 30 minutes, the addition was complete, the solution was adjusted to pH 4.75 with ACOH, and mixed with the resin "IRA-45" (Cl, 200 ml), and the mixture was stirred slowly. The solution was passed through a column of the resin "IRA-45" (Cl-, 100 ml). The eluent thus obtained was passed through a column of the resin "HP-20" for adsorption. The column was washed with 1% AcOH and then eluted with CH$_3$CN/H$_2$O/AcOH (8/1/1), and the eluent thus obtained was concentrated and freeze-dried. The powder thus obtained was purified by column chromatography with CM-cellulose, using gradient elution, and as an eluting agent 0.05M → 0.7M NH$_4$Ac (1l → 1l). The main fractions (fractions 89 to 99) were collected, combined and freeze-dried. The powder thus obtained was purified by the resin "HP-20" and then by the resin "LH-20". A part (10 mg) of the thus purified powder was dissolved in 1N ACOH (3 ml), and H$_2$O$_2$ (0.5 ml) was added thereto. The solution was stirred for 20 minutes and then contacted with the resin "HP-20" for adsorption. The resin was washed with 1% AcOH and eluted with CH$_3$CN/H$_2$O/AcOH (8/1/1). The eluent thus obtained was concentrated and freze-dried to obtain Met(O)$^{12}$-hANP (1—28) in a yield of 6 mg.

HPLC [column: nucleosil 5 (18; 0.1% TFA, CH$_3$CN 1% → 60% gradient elution]

Single peak, 24.8 minutes

Amino acid analysis [Hydrolysis with 6N HCl]

Arg: 1.00 × 5, Asp: 1.00 × 2, Ser: 0.91 × 5, Glu: 1.01, Gly: 1.02 × 5, Ala: 1.06, 1/2(Cys)$_2$: 0.88 × 2, Met: 0.89, Ile: 0.92, Leu: 1.05 × Tyr: 0.89, Phe: 1.00 × 2.

## Example 8
### Synthesis of [Met$^{12}$, Asu$^{7,23}$]-α-hANP (7—28)

(1) Synthesis of Boc-Ala-Gln-Ser(Bzl)-Gly-Leu-Gly-Asu(OPac)-Asn-Ser(Bzl)-Phe-Arg(Tos)-Tyr(Cl$_2$Bzl)-OBzl

Boc-Asu(OPac)-Asn-Ser(Bzl)-Phe-Arg(Tos)-Tyr(Cl$_2$Bzl)-OBzl (0.95 g, 0.60 mmole) was reacted with CF$_3$CO$_2$H (4 ml, 70 mole) for 20 minutes with cooling and for 40 minutes at room temperature. Then, 5.9N HCl in dioxane (0.2 ml, 1.5 mole) was added thereto and the excess acid was distilled off. To the residue ether was added and the powder thus obtained was dried under reduced pressure over NaOH. This powder, together with Boc-Ala-Gln-Ser(Bzl)-Gly-Leu-Gly-OH (0.455 g, 1.05 mole), was dissolved in a mixture of N-methylpyrolidone/DMF (2:1), and WSCl (0.112 ml, 1.1 mole) was added dropwise thereto with stirring and cooling to −20°C (pH about 5). The mixture was stirred overnight, and it then had a negative response in a fluorescamine test. To the reaction solution water was added, and the solid material thus precipitated was recovered by filtration, and washed with water and then with ether. It was reprecipitated from chloroform-methanol/ether to obtain the desired product in a yield of 0.88 g (68%).

Amino acid analysis [Hydrolysis with 6N HCl, 110°C, 22 hour addition of phenol]

$NH_3$: 1.16 × 2, Arg: 0.94, Asp: 1.00, Ser: 0.90 × 2, Glu: 0.98, Gly: 0.98 × 2, ala: 1.00, Asu: 1.02, Leu: 0.98, Tyr: 0.91, Phe: 0.97

(2) Synthesis of Boc-Ala-Gln-Ser(Bzl)-Gly-Leu-Gly-Asu(OH)-Asn-Ser(Bzl)-Phe-Arg(Tos)-Tyr(Cl₂Bzl)-OBzl

Boc-Ala-Gln-Ser(Bzl)-Gly-Leu-Gly-Asu(OPac)-Asn-Ser(Bzl)-Phe-Arg(Tos)-Tyr(Cl₂Bzl)-OBzl (0.84 g, 0.387 mmole) was dissolved in $CH_3COOH$ (60 ml), and stirred while heating to about 45°C for 1 hour in the presence of zinc powder.

The catalyst was removed by filtration and $CH_3COOH$ was distilled off. To the residue water was added and the solid material thus precipitated was recovered by filtration, and washed with water and with ether. It was reprecipitated from chloroform-methanol/ether to give the required product in a yield of 0.68 g (86%).

(3) Synthesis of Boc-Phe-Gly-Gly-Arg(Tos)-Met-Asp(OChx)-Arg(Tos)-Ile-Gly-OPac

Boc-Arg(Tos)-Met-Asp(OChx)-Arg(Tos)-Ile-Gly-OPac (1.35 g, 1.0 mmole) was reacted with $CF_3CO_2H$ (6 ml, 100 mole) for 20 minutes with cooling and for 40 minutes at room temperature. Then, 5.9N HCl/dioxane (0.21 ml, 1.2 mole) was added thereto, and the excess acid was distilled off. To the residue ether was added and the powder thus obtained was recovered by filtration and dried under reduced pressure over NaOH.

This powder, Boc-Phe-Gly-Gly-OH (0.40 g, 1.05 mole) and HOBt (0.15 g, 1.1 mole) were dissolved in a mixture (15 ml) of N-methylpyrolidone and DMF (1:2). WSCI (0.201 ml, 1.1 mole) was added thereto with cooling to −20°C and stirring (pH about 5).

The next day, it had a negative response in a fluorescamine test. The reaction solution was poured into water and the solid material thus precipitated was recovered by filtration and washed with water and ether. It was reprecipitated from CHCl₃-MeOH/ether to give the required product (1.48 g, 92%).

(4) Synthesis of Boc-Ala-Gln-Ser(Bzl)-Gly-Leu-Gly-Asu-(Phe-Gly-Gly-Arg(Tos)-Met-Asp(OChx)-Arg(Tos)-Ile-Gly-OPac)-Asn-Ser(Bzl)-Phe-Arg(Tos)-Tyr(Cl₂Bzl)-OBzl

Boc-Phe-Gly-Gly-Arg(Tos)-Met-Asp(OChx)-Arg(Tos)-Ile-Gly)OPac (0.14 g, 86.5 micromole) was reacted with $CF_3CO_2H$ (1 ml, 100 mole) for 20 minutes with cooling and for 40 minutes at room temperature. Then 5.9N HCl/dioxane (0.1 ml) was added thereto, and the excess acid was distilled off. To the residue ether was added and the powder thus obtained was recovered by filtration, and dried under reduced pressure over NaOH.

The powder, together with Boc-Ala-Gln-Ser(Bzl)-Gly-Leu-Gly-Asu-Asn-Ser(Bzl)-Phe-Arg(Tos)-Tyr(Cl₂Bzl)OBzl (0.18 g, 1.05 mole) and HOBt (15 mg, 1.1 mole) was dissolved in N-methylpyrolidone/DMF (5:1, 6 ml). WSCI (187 microlitres, 1.1 mole) was added thereto with cooling to −20°C and stirring (pH about 5). On the following day, to the reaction solution, water was added, and the solid material thus precipitated was recovered by filtration, washed with water and ether, and reprecipitated from DMF/ether to give the required product (0.25 g, 81%).

Amino acid analysis

$NH_3$: 1.22 × 2, Arg: 0.96 × 3, Asp: 1.00 × 2, Ser: 0.98 × 2, Glu: 1.10, Gly: 0.98 × 5, Ala: 1.10, Met: 0.66, Asu: 1.10, Ile: 0.89, Leu: 1.17, Tyr: 1.04, Phe: 0.99 × 2.

(5) Synthesis of Boc-Ala-Gln-Ser(Bzl)-Gly-Leu-Gly-Asu(Phe-Gly-Gly-Arg(Tos)-Met-Asp(OChx)-Arg(Tos)-Ile-Gly-OH)-Asn-Ser(Bzl)-Phe-Arg(Tos)-Tyr-Tyr(CL₂Bzl)-OBzl

Boc-Ala-Gln-Ser(Bzl)-Gly-Leu-Gly-Asu(Phe-Gly-Gly-Arg(Tos)-Met-Asp(OChx)-Arg(Tos)-Ile-Gly-OPac)-Asn-Ser(Bzl)-Phe-Arg(Tos)-Tyr(CL₂Bzl)-OBzl (0.22 g, 61.9 micromole) was dissolved in $CH_3CO_2H$ (10 ml), and stirred for 1 hour in the presence of zinc powder (1.0 g) while heating to 45°C. The catalyst was removed by filtration and $CH_3CO_2H$ was distilled off. To the residue water was added and the thus precipitated solid material was recovered by filtration, washed with water and ether, and reprecipitated with DMF/ether to give the desired product (0.20 g, 95%).

(6) Synthesis Phe-Gly-Gly-Arg(Tos)-Met-Asp(OChx)-Arg(Tos)-Ile-Gly-Ala-Gln-Ser(Bzl)-Gly-Leu-Gly-Asu-Asn-Ser(Bzl)-Phe-Arg(Tos)-Tyr(Cl₂Bzl)-OBzl

Boc-Ala-Gln-Ser(Bzl)-Gly-Leu-Gly-Asu-(Phe-Gly-Gly-Arg(Tos)-Met-Asp(OChx)-Arg(Tos)-Ile-Gly-OH)-Asn-Ser(Bzl)-Phe-Arg(Tos)-Tyr(Cl₂Bzl)-OBzl (0.19 g, 55.3 micromole) was reacted with $CF_3CO_2$ (1 ml, 220 mole) for 10 minutes with cooling and for 50 minutes at room temperature. Then 5.9N HCl/dioxane (20 microlitres, 2 mole) was added thereto, and the excess acid was distilled off. To the residue ether was added, and the powder thus precipitated was recovered by filtration and dried under reduced pressure over NaOH.

The powder and HOBt (15 mg, 2 mole) were dissolved in DMF (30 ml) and WSCI (11.1 microlitres, 1.1 mole) was added thereto with cooling to −20°C and stirring (pH 4 to 5). After 2 hours, WSCI·HCl (11 mg, 1 mole) was further added. Next day, the solution had a negative response in a fluorescamine test. DMF was distilled off. To the residue, water was added. The solid material thus precipitated was recovered by filtration, washed with water and ether, and reprecipitated from DMF/ether to give the required product (0.15 g, 82%).

Amino acid analysis

NH$_3$: 1.27, Arg: 0.96 × 3, Asp: 1.00 × 2, Ser: 0.98 × 2, Glu: 1.10, Gly: 0.97 × 5, Ala: 1.11, Met: 0.63, Asu: 1.12, Ile: 0.89, Leu: 1.16, Tyr: 1.01, Phe: 0.99 × 2.

(7) Synthesis of deamino [Met$^{12}$, Asu$^{7,23}$]-α-hANP (5—28)

Protected and ring-forming [Met, Asu] ANP (7—28) (130 mg, 39.1 micromole) was reacted with HF (5 ml) in the presence of anisole (0.4 ml, 80 mole) for 1 hour with cooling. HF was distilled off, and the residue was dissolved in 50% CH$_3$CO$_2$H. An aqueous layer thereof was washed with ether and passed through a column of the resin "Dowex 1 × 2; Aco⁻, 30 ml). The column was eluted with 1N CH$_3$CO$_2$H, and the fractions having a positive response in a Pauly test were collected, combined together and freeze-dried. The product was further purified as follows:

(1) CM-cellulose (2.1 cm diameter × 22 cm) Column Purification:
linear gradient elution with 0.03M AcONH$_4$ (pH 4.8) → 0.3M (each 300 ml); yield: 40 mg.

(2) "HP-20" (2.1 cm diameter × 23 cm) Column Purification
linear gradient elution with 0% → 30% CH$_3$CN/1N AcOH (each 400 ml); yield: 12 mg (13%)

Amino acid analysis

NH$_3$: 1.89 × 2, Arg: 0.99 × 3, Asp: 0.99 × 2, Ser: 0.88 × 2, Glu: 0.98, Gly: 1.00 × 5, Ala: 1.02, Met: small to middle peak, Asu 1.05, Ile: 1.05, Leu: 1.14, Tyr: 0.93, Phe: 0.99 × 2.

Example 9

Synthesis of [Ile$^{12}$, Asu$^{7,23}$]-α-hANP (7—28)

(1) Synthesis of Boc-Phe-Gly-Gly-Arg(Tos)-Ile-Asp-(OChx)-Arg(Tos)-Ile-Gly-OPac

The required product (0.61 g, 95%) was obtained in the same manner as in Example 8 (1) using as starting materials Boc-Arg(Tos)-Ile-Asp(OChx)-Arg(Tos)-Ile-Gly-OPac (0.535 g, 0.40 mmole) and Bo-Phe-Gly-Gly (0.16 g, 1.05 mole).

Amino acid analysis

Arg: 0.98 × 2, Asp: 1.03, Gly: 1.00 × 3, Ile: 0.98 × 2, Phe: 0.99

(2) Synthesis of Boc-Ala-Gln-Ser(Bzl)-Gly-Leu-Gly-Asu(Phe-Gly-Gly-Arg(Tos)-Ile-Asp(OChx)-Arg(Tos)-Ile-Gly-OPac)-Asn-Ser(Bzl)-Phe-Arg(Tos)-Tyr(Cl$_2$Bzl)-OBzl

The required product (0.62 g, 91%) was obtained in the same manner as in Example 8 (4) using as starting materials the peptide as mentioned above in step (1) (0.31 g, 0.194 mmole) and Boc-Ala-Gln-Ser(Bzl)-Gly-Leu-Gly-Asu(OH)-Asn-Ser(Bzl)-Phe-Arg(Tos)-Tyr(Tos)-OBzl (0.406 g, 1.02 mole).

(3) Synthesis of Boc-Ala-Gln-Ser(Bzl)-Gly-Leu-Gly-Asu(Phe-Gly-Gly-Arg(Tos)-Ile-Asp(OChx)-Arg(Tos)-Ile-Gly-OH)-As-Ser(Bzl)-Phe-Arg(Tos)-Tyr(C$_2$Bzl)-OBzl

The required product (0.55 g, 94%) was obtained in the same manner as in Example 8 (5) using as a starting material the peptide as mentioned above in step (2) (0.60 g, 0.17 mmole).

(4) Synthesis of Phe-Gly-Gly-Arg(Tos)-Ile-Asp(OChx)-Arg(Tos)-Ile-Gly-Ala-Gln-Ser(Bzl)-Gly-Leu-Gly-Asu-Asn-Ser(Bzl)-Phe-Arg(Tos)-Tyr(Cl$_2$Bzl)-OBzl

The required product (0.44 g, 88%) was obtained in the same manner as in Example 8 (6) using as a starting material the peptide as mentioned above in step (3) (0.51 g, 0.15 mmole).

Amino acid analysis

NH$_3$: 1.28 × 2, Arg: 0.92 × 3, Asp: 1.00 × 2, Ser: 0.91 × 2, Glu: 1.01, Gly: 0.97 × 5, Ala: 1.03, Asu: 0.96, Ile: 0.85 × 2, Leu: 0.93, Tyr: 0.95, Phe: 0.94 × 2.

(5) Synthesis of Deamino [Ile$^{12}$, Asu$^{7,23}$]-α-ANP (7—28)

The required product (20 mg, 8% in purified form) was obtained in the same manner as Example 8 (7) using as a starting material the peptide as mentioned above in step (4) (0.40 g, 0.12 mmole).

Amino acid analysis

NH$_3$: 1.23 × 2, Arg: 0.97 × 3, Asp: 1.00, Ser: 0.95 × 2, Glu: 0.96, Gly: 1.00, Ala: 1.02, Asu: 1.06, Ile: 1.02 × 2, Leu: 1.03, Tyr: 0.93, Phe: 0.97 × 2

24

## Example 10

Synthesis of [Nle$^{12}$, Asu$^{7,23}$]-α-ANP (7—28)

(1) Synthesis of Boc-Phe-Gly-Gly-Arg(Tos)-Nle-Asp(OBzl)-Arg(Tos)-Ile-Gly-OPac

The required product (0.37 g, 79%) was obtained in the same manner as in Example 8 (1) using as starting materials Boc-Arg(Tos)-Nle-Asp(OBzl)-Arg(Tos)-Ile-Gly-OPac (0.40 g, 0.297 mmole) and Boc-Phe-Gly-Gly (0.117 g, 1.05 mole).

(2) Synthesis of Boc-Ala-Gln-Ser(Bzl)Gly-Leu-Gly-Asu(Phe-Gly-Gly-Arg(Tos)-Nle-Asp(OBzl)-Arg(Tos)-Ile-Gly-OPac)-Asn-Ser(Bzl)-Phe-Arg(Tos)-Tyr(Cl$_2$Bzl)-OBzl

The required product (0.364 g, 85%) was obtained in the same manner as in Example 8 (4) using as starting materials the peptide as mentioned above in step (1) (0.195 g, 0.121 mmole), and Boc-Ala-Gln-Ser(Bzl)-Gly-Leu-Gly-Asu(OH)-Ser(Bzl)-Phe-Arg(Tos)-Tyr(Cl$_2$Bzl)-OBzl (0.254 g, 1.02 mole).

Amino acid analysis

NH$_3$: 1.23 × 2, Arg: 0.94 × 3, Asp: 1.00 × 2 Ser: 0.94 × 2, Glu: 1.04, Gly: 0.99 × 5 Ala: 1.04, Nle: 0.96, Asu: 1.12, Ile: 0.92, Leu: 1.11, Tyr: 1.02, Phe: 0.96 × 2.

(3) Synthesis of Boc-Ala-Gln-Ser(Bzl)-Gly-Leu-Gly-Asu-(Phe-Gly-Gly-Arg(Tos)-Nle-Asp(OBzl)-Arg(Tos)-Ile-Gly-OH,Asn-Ser(Bzl)-Phe-Arg(Tos)-Tyr-Cl$_2$Bzl)-OBzl

The required product (0.29 g, 89%) was obtained in the same manner as in Example 8 (5) using as a starting material the peptide as produced in step (2) above (0.34 g, 96 micromole).

(4) Synthesis of Phe-Gly-Gly-Arg(Tos)-Nle-Asp(OBzl)-Arg(Tos)-Ile-Gly-Ala-Gln-Ser(Bzl)-Gly-Leu-Gly-Asu-Asn-Ser(Bzl)-Phe-Arg(Tos)-Tyr(Cl$_2$Bzl)-OBzl

The required product (0.22 g, 88%) was obtained in the same manner as in Example 8 (6) using as a starting material the peptide as produced in the above step (3) (0.26 g 75.9 micromole).

Amino acid analysis

NH$_3$: 1.31 × 2, Arg: 0.94 × 3, Asp: 1.00 × 2 Ser: 0.94 × 2, Glu: 1.05, Gly: 98 × 5, Ala: 1.03, Nle: 0.99, Asu: 1.16, Ile: 0.97, Leu: 1.14, Tyr: 1.03, Phe: 96 × 2.

(5) Synthesis of Deamino [Nle$^{12}$, Asu$^{7,23}$]-α-ANP (7—28) The required product (28 mg, 20%) in purified form was obtained in the same manner as in Example 8 (7) using as a starting material the peptide as mentioned in the above step (4) (0.20 g, 0.060 mmole).

Amino acid analysis

NH$_3$: 1.33 × 2, Arg: 1.00 × 3, Asp: 1.02 × 2, Ser: 0.90 × 2, Glu: 0.98, Gly: 1.00 × 5, Ala: 1.00, Nle: 0.99, Asu: 1.08, Ile: 1.02, Leu: 1.03, Tyr: 0.95, Phe: 0.98 × 2.

## Example 11

Synthesis of (Nle$^{12}$)-α-hANP (7—28)

(1) Synthesis of Protected (Nle$^{12}$)-α-hANP (7—28)

To Boc-Arg(Tos)-Nle-Asp(OBzl)-Arg(Tos)-Ile-Gly-Ala-Gln-Ser(Bzl)-Gly-Leu-Gly-Cys(4CH$_3$Bzl)-Asn-Ser-(Bzl)-Phe-Arg(Tos)-Tyr(Cl$_2$Bzl)-OBzl (650 ml, 0.20 mmole), TFA (5 ml) was added and stirred for 50 minutes. TFA was distilled off, and to the residue 3.5N HCl/dioxane (114 microlitres, 0.4 mmole) was added and stirred well. Ether was added thereto. The solid material thus precipitated was recovered by filtration, dried and dissolved in N-methylpyrolidone (15 ml). HOBt (36 mg), Boc-Cys(4CH$_3$Bzl)-Phe-Gly-Gly-OH (153 mg) and WSCI (48 microlitres) were added thereto while cooling to −15°C. The solution was stirred for 16 hours and had a negative response in a fluorescamine test. To the reaction solution thus gelatinized, water was added, and the precipitate thus produced was recovered by filtration and refluxed twice with methanol to the required product in a yield of 700 mg (95.4%).

Amino acid analysis

NH$_3$: 2.02, Arg: 0.88 × 3, Asp: 1.00 × 2, Ser: 0.89 × 2, Glu: 1.00, Gly: 1.02 × 5, Ala: 1.03, 1/2(Cys)$_2$: small peak, Nle: 0.88, Ile: 0.89, Leu: 0.97, Tyr: 0.87, Phe: 1.01 × 2.

(2) Synthesis of (Nle$^{12}$)-α-hANP (7—28)

The protected (Nle$^{12}$) α-hANP (7—28) (650 mg, 0.177 mmole) was treated with anisole (1 ml) and HF (10 ml) at 0°C for 60 minutes. HF was distilled off and to the residue ether was added. The precipitate thus produced was washed well with ether and dissolved in 1N CH$_3$CO$_2$H. The solution was passed through a column of the resin "Dowex 1 × 2" (AcO$^-$). The column was eluted with 1N CH$_3$CO$_2$H. The eluent obtained was dried, to give a powder, dissolved in 1N CH$_3$CO$_2$H (18 ml). This solution was added dropwise to a mixture of 1M NH$_4$OAc/6M urea solution (162 ml) and K$_3$Fe(CN)$_6$ (83 mg). The pH was kept at 7.4 with 10% NH$_4$OH. After the addition, the solution was stirred for 30 minutes, and was then adjusted to pH 4.75 with CH$_3$CO$_2$H. The solution was then passed through a column of IRA-45 (Cl$^-$, 100 ml). The column was washed with CH$_3$CO$_2$H. The washing liquid was desalted with the resin "Diaion HP-20" and eluted with acetonitrile/water/CH$_3$CO$_2$H (8/1/1). The eluent was concentrated and freeze-dried. The powder thus obtained was purified by chromatography (CM-Cellulose; gradient elution; 0.05 → 0.4M NH$_4$OAc). Fractions 50 to 57 were collected, combined together and freeze-dried. The powder obtained was further purified by chromatography (resin "Diaion HP-20"; gradient elution; eluting solvent: 5% $CH_3CN \rightarrow$ 25% $CH_3CN$/5% $CH_3CO_2H$). Fractions 75 to 87 were collected, combined together and freeze-dried. Next, the product was desalted with Sephadex LH-20, and eluted with 2N $CH_3CO_2H$. Fractions 11 to 15 were collected, combined together and freeze-dried to give the required purified product in a yield of 64 mng.

The product has one single peak at 25.5 minutes when examined by HPLC using a column of Nucleosile $5C_{18}$ and 1 to 60% $CH_3CN$/0.1% TPA as an eluting solution.

Amino acid analysis

$NH_3$: 2.57, Arg: 1.03 × 3, Asp: 1.01 × 2, Ser: 0.91 × 2, Gln: 0.9, Gly: 1.00 × 5, Ala: 1.01, 1/2(Cys)$_2$: 0.86 × 2, Nle: 0.93, Ile: 0.9, Leu: 0.96, Tyr: 0.81, Phe: 1.00.

Example 12

Synthesis of α-hANP (4—28)

(1) Synthesis of Boc-Arg(Tos)-Ser(Bzl)-Ser(Bzl)-Cys(4-CH$_3$Bzl)-Phe-Gly-Gly-OH

Boc-Arg(Tos)-Ser(Bzl)-Ser(Bzl)-Cys(4CH$_3$Bzl)-Phe-Gly-Gly-OPac (2 g, 1.46 mmole) was dissolved in $CH_3CO_2H$ (100 ml), and zinc powder (5 g) was added thereto. The mixture was stirred for 50 minutes at 45°C. The Zm-powder was removed by filtration and $CH_3CO_2H$ was distilled off. To the residue water was added and the precipitate thus produced was recovered by filtration and then recrystallized from methanol to the required product (1.5 g, 82.0%).

Amino acid analysis

Arg: 0.98, Ser: 0.87 × 2, Gly: 1.00 × 2, 1/2(Cys)$_2$: small peak, Phe: 1.00.

(2) Synthesis of protected α-hANP (4—28)

Boc-Arg(Tos)-Ser(Bzl)-Ser(Bzl)-Cys(4-CH$_3$Bzl)-Phe-Gly-Gly-Arg(Tos)-Met-Asp(OBzl)-Arg(Tos)-Ile-Gly-Ala-Gln-Ser(Bzl)-Gly-Leu-Gly-Cys(4-CH$_3$Bzl)-Asn-Ser(Bzl)-Phe-Arg(Tos)-Tyr(Cl$_2$Bzl)-OBzl

Boc-Arg(Tos)-Met-Asp(CHx)-Arg(Tos)-Ile-Ala-Gln-Ser(Bzl)-Gly-Leu-Gly-Cys(4-CH$_3$Bzl)-Asn-Ser(Bzl)-Phe-Arg-Tyr(CL$_2$Bzl)-OBzl (0.644 g, 0.20 mmole) was treated with $CF_3CO_2H$ (3 ml) for 10 minutes with cooling to −5°C and for 50 minutes at room temperature. 5.9N HCl/dioxane (60 microlitres, 1.5 mole) was added thereto and the excess acid was distilled off. To the residue ether was added. The powder obtained was dried over NaOH.

The powder thus obtained, Boc-Arg(Tos)-Ser(Bzl)-Cys(4CH$_3$Bzl)-Phe-Gly-Gly-OH (0.263 g, 1.05 mole) and HOBt (30 mg, 1.1 mole) were dissolved in DMF (4 ml) and N-methylpyrolidone (4 ml) and WSCl (40.3 microlitres, 1.1 mole) was added thereto with cooling so that the temperature did not exceed 20°C and with stirring. The pH of the reaction solution was about 6.

On the following day, it had a negative response in a fluorescamine test. Into the precipitated gelatinous material, water was poured. The solid material thus produced was recovered by filtration and washed with water, n-hexane and ether in the order given.

The product was suspended in DMF, and methanol was added thereto. The powder thus produced was recovered, by filtration and washed with methanol to give the required product (0.79 g, 91%).

Amino acid analysis

$NH_3$: 1.27 × 2, Arg: 0.89 × 4, Asp: 1.00 × 2, Ser: 0.88 × 4, Glu: 1.07, Gly: 1.00 × 5, Ala: 1.03, 1/2(Cys)$_2$: 0.18 × 2, Met: 0.29 Ile: 0.90, Leu: 0.98, Tyr: 0.96, Phe: 0.97 × 2.

(3) Synthesis of α-hANP (4—28)

The protected peptide α-hANP (4—28) (0.61 g, 0.14 mmole) was reacted with $CF_3CO_2H$ (3 ml) for 10 minutes with cooling to −5°C and for 50 minutes at room temperature. Then 5.9N HCl/dioxane (60 microlitres) was added thereto. The excess acid was distilled off, and to the residue ether was added. The powder thus produced was recovered and dried over NaOH.

The powder was treated with anhydrous HF (about 8 ml) in the presence of anisole (1.1 ml) for 1 hour with cooling. HF was distilled off with cooling. The residue thus obtained was dissolved in 50% $CH_3CO_2H$, diluted with water and washed with ether. The aqueous phase was passed through a column of the resin "Dowex 1 × 2" (AcO$^-$, 40 ml). The column was eluted with 1N $CH_3CO_2H$. The fractions having a positive response in a Pauly test were collected, combined together and freeze-dried.

The freeze-dried product was dissolved in 1N $CH_3CO_2H$ (20 ml) containing urea. The solution was added dropwise to 1M $AcONH_4$ (pH 7.4)/8M urea (120 ml) containing $K_3Fe(CN)_6$ (65 mg, 1.4 mole) over about 10 minutes. Meanwhile, the pH was kept at 7.4 by the addition of 10% ammonia. The solution was further stirred for 10 minutes and then adjusted to pH 4 to 5 with $CH_3CO_2H$. The resin "IRA-45" (Cl$^-$, 30 ml) was added thereto and the mixture was stirred slowly. Next, the solution was passed through a column of the resin "IRA-45" (Cl$^-$, 30 ml) and then of the resin "HP-20" (fine, 50 ml). The column was washed with $CH_3CO_2H$ (200 ml) and then eluted with acetonitrile/$CH_3CO_2H$/water (8/1/1) (300 ml) from the column of "HP-20". The eluent was concentrated and freeze-dried with 1N $CH_3CO_2H$. It was further purified as follows:

(1) CM-Cellulose Column (2.1 cm diameter × 28 cm) Purification

Linear gradient elution using 0.06M $AcONH_4$ (pH 4.8) → 0.6M-$AcONH_4$ (pH 4.8) (each 400 ml); yield: 100 mg.

26

# EP 0 173 557 B1

(2) "HP-20" Column (2.4 cm diameter × 22 cm) Purification

Linear gradient elution using 0% $CH_3CN$/1% $CH_3CO_2H$ 25% $CH_3CN$/1% $CH_3CO_2H$ (each 400 ml); yield: 60 mg.

(3) "LH-20" Column (2.13 cm diameter × 64 cm) purification

Elution with 1N $CH_3CO_2H$ to give the purified product (36 mg, 9.5%).

Amino acid analysis

$NH_3$: 1.28 × 2, Arg: 1.03 × 4, Asp: 1.00, Ser: 0.91 × 4, Glu: 1.00, Gly: 1.00 × 5, Ala: 1.01, 1/2 $(Cys)_2$: 0.82 × 2, Met: 0.81, Ile: 0.90, Leu: 0.97, Tyr: 0.97, Phe: 1.00 × 2.

## Example 13

Synthesis of α-hANP (7—27)

Boc-Arg(Tos)-Met-Asp(OChx)-Arg(Tos)-Ile-Gly-Ala-Gln-Ser(Bzl)-Gly-Leu-Gly-Cys(4CH₃Bzl)-Asn-Ser(Bzl)-Phe-Arg(Tos)-OBzl (724 mg, 10.25 mm) was treated with TFA (10 ml) for 10 minutes with cooling and for 40 minutes at room temperature. Then, 5.9N HCl/dioxane (0.2 ml, 5 mole) was added thereto. The solvent and excess acid were distilled off. To the residue Et₂O was added and the powder thus produced was recovered by filtration and dried overnight under reduced pressure over NaOH.

The powder, together with Boc-Cys(4CH₃Bzl)-Phe-Gly-Gly-OH (154 mg, 1.05 mole) and HOBt (37 mg, 1.1 mole) was dissolved in N-methyl pyrolidone (NMP) (10 ml), and WSCI (50 microlitres, 1.1 mole) was added dropwise thereto with cooling and solution was stirred overnight. On the next day, the solution thus gelatinized had a negative response in a fluorescamine test.

To the solution water was added and the solution was filtered to obtain solid material. The solid material was washed with water, n-hexane and Et₂O in the order given, and dried. It was suspended in DMF and MeOH was added thereto. The solution was filtered. The solid material obtained by filtration was dried over P₂O₅ to give protected and non-cyclized ANP (7—27) in a yield of 735 mg (87.4%).

Amino acid analysis

Arg: 0.97 × 3, Asp: 0.99 × 2, Ser: 0.87 × 2, Glu: 1.02; Gly: 0.99 × 2, Ala: 1.02, Cys: 0.34 × 2, Met: 0.82, Ile: 0.92, Leu: 1.00, Phe: 0.97 × 2.

Elementary analysis

Found:       C 57.03%,   H 6.38%,   N 13.08%

Calculated:   C 57.02%,   H 6.39%,   N 13.22%

(for $C_{161}H_{212}O_{36}N_{32}S_6$·1.5H₂O).

The peptide ocntaining the protecting groups, as described above (437 mg, 0.13 mmole) was treated with TFA (5 ml) for 60 minutes and 5N HCl/dioxane (0.1 ml) was added thereto, and the solvent was distilled off. To the residue Et₂O was added and the precipitated powder was recovered by filtration, and dried overnight over NaOH. The powder was treated with HF (7 ml) in the presence of anisole (1.25 ml) while cooling to −2 to −1°C for 60 minutes. The excess HF was distilled off. The residue was dissolved in 30% AcOH and washed three times with Et₂O, and passed through a column of the resin "Dowex 1 × 2" (AcO⁻, 45 ml). The column was eluted with 1N AcOH, and the fractions having a positive response in a Pauli test were collected, combined together and freeze-dried. The 2SH derivative thus obtained was connected to the disulphide derivative by a conventional cyclization method. The product was purified by (1) CM-Cellulose column chromatography (eluting agent 0.05M (pH 4.7) → 0.45 (pH 4.8) NH₄OAc), (2) "HP-20" column chromatography (eluting agent 0 → 23% $CH_3CN$/5% AcOH), and (3) Sephadex "LH-20" column chromatography (eluting agent 0.5N AcOH) to give the required purified product (29 mg).

Amino acid analysis

Arg: 1.04 × 3, Asp: 1.00 × 2, Ser: 0.90 × 2, Glu: 0.97, Gly: 1.00 × 5, Ala: 1.00, Cys: 0.87 × 2, Met: 0.86, Ile: 0.95, Seu: 0.97, Phe: 1.00 × 2,

Elementary analysis

Found:       C 46.37%,   H 6.69%,   N 18.00%

Calculated:   C 46.23%,   H 6.74%,   N 18.16%,

(for $C_{91}H_{144}O_{28}N_{32}S_3$·2AcOH.8H₂O)

## Example 14

Synthesis of [D-Ala₉]α-hANP (4—28)

(1) Synthesis of Boc-D-Ala-Gly-OBzl

Boc-D-Ala-OH (5.68 g, 30 mmole) and H-Gly-OBzl·-TosOH (10.6 g, 1.05 eq) were suspended in CH₂Cl₂, and WSCI (6 ml, 1.1 eq) was added dropwise thereto with cooling, and the solution was stirred overnight. The CH₂Cl₂ was distilled off and the residue was dissolved in AcOEt (500 ml), washed with 1N HCl, H₂O, 5% NaHCO₃, and H₂O in the order given, and dried over Na₂SO₄. AcOEt ws distilled off and the residue was recrystallized twice from AcOEt/n-hexane to give the desired product (9.16 g, 90.7%).

(2) Synthesis of Boc-Phe-D-Ala-Gly-OBzl

Boc-D-Ala-Gly-OBzl (3.36 g, 10 mmole) was stirred with TFA (10 ml) for 10 minutes with cooling and for 30 minutes at room temperature. 5.0N HCl/dioxane (2.4 ml, 1/2 eq) was added thereto, the solvent was

distilled off and Et$_2$O-n-hexane was added to the residue to give an oily substance. After decanatation, the resulting substance was dried over NaOH for 3 hours. The substance obtained, together with Boc-Phe (2.65 g, 1eq) and HOBt (1.42 g, 1.05 eq) was dissolved in DMF (12 ml), and WSCl (1.92 ml, 1.05 eq) was added dropwise thereto with cooling, and the solution was stirred overnight. To the solution AcOEt (150 ml) was added and the solution was washed with 1N HCl, H$_2$O 5% NaHCO$_3$ and H$_2$O in the order given, and then dried over Na$_2$SO$_4$. AcOEt was distilled off and the residue was recrystallized from AcOEt/Et$_2$O and MeOH/Et$_2$O to give the desired product (2.57 g).

(3) Synthesis of Boc-Phe-D-Ala-Gly-OH

Boc-Phe-D-Ala-Gly-OBzl (2.4 g, 4.96 mmole) was dissolved in MeOH (50 ml) and H$_2$ gas was passed through the solution in the presence of Pd-C for 4 hours. The Pd-C was removed by filtration and MeOH was distilled off. To the residue thus obtained MeOH/Et$_2$O and n-hexane were added. The powder thus precipitated was recovered by filtration and reprecipitated from AcOEt/n-hexane to give the desired product (1.95 g, 100%).

(4) Synthesis of Z-Cys(4-CH$_3$Bzl)-Phe-D-Ala-Gly-OH

Boc-Phe-D-Ala-Gly-OH (1.58 g, 4 mmole) was treated with TFA (10 ml), for 10 minutes with cooling and for 30 minutes at room temperature. The excess TFA was distilled off. To the residue, Et$_2$O was added. The powder thus produced was recoevred by filtration and dried for 6 hours over NaOH. It was suspended in a mixture of DMF (5 ml)/NMP (30 ml), and NEt$_3$ (0.56 ml) was added thereto with cooling. Thereafter, Z-Cys-(4-CH$_3$Bzl)-OSu (2 g, 1.1 eq) was added and the mixture was stirred overnight. This transparent solution had a negative response in a fluorescamine test, and was poured into dilute aqueous HCl. The powder thus produced was recovered by filtration, washed with H$_2$O and n-hexane, dissolved in a mixture of MeOH and CHCl$_3$, dehydrated by flashing with toluene, and reprecipitated with AcOEt from E$_2$O and from n-hexane to give a powder of the required product (1.86 g, 72.4%).

Amino acid analysis
Gly: 0.99, Ala: 1.00, Cys: small peak, Phe: 1.00
Elementary analysis
Found:     C 61.88%,  H 6.04%,  N 9.03%
Calculated:  C 62.00%,  H 6.07%,  N 8.76%,
(for C$_{33}$H$_{38}$O$_7$N$_4$S$\cdot$1/4H$_2$O)

(5) Synthesis of Protected [D-Ala$^9$]-α-hANP (7—28)

Boc-Arg(Tos)-Met-Asp(OChx)-Arg(Tos)-Ile-Gly-Ala-Gln-Ser(Bzl)-Gly-Leu-Gly-Cys(4CH$_3$Bzl)-Asn-Ser(Bzl)-Pe-ARg(Tos)-Tyr(Cl$_2$Bzl)-OBzl (644 mg, 0.2 mmole) was stirred with TFA (5 ml) for 10 minutes with cooling and for 50 minutes at room temperature. 5.0N HCl/dioxane (0.1 ml) was added thereto. The solvent was distilled off, and to the residue Et$_2$O was added to give a powder., The powder was recovered by filtration and dried overnight over NaOH. The power, together with Z-Cys(4CH$_3$Bzl)-Phe-D-Ala-Gly-OH (133 mg, 1.05 eq) and HOBt (30 mg, 1.1 eq), was dissolved in NMP (8 ml), WSCl (4 microlitres, 1.1 eq) was added thereto with cooling, and the mixture was stirred for 6 hours. To the gelatinous solution, which had a negative response in a fluorescamine test, water was added. The solution thus obtained was filtered and the solid material obtained was washed with H$_2$O, n-hexane, Et$_2$O and MeOH in the order given, and dried. The material was suspended in DMF and MeOH was added thereto. The solution was filtered. The solid material obtained was washed with MeOH to give the required product (640 mg, 85.7%).

Amino acid analysis
Arg: 1.01 × 3, Asp: 1.00 × 2, Ser: 0.89 × 2 Glu: 1.01, Gly: 1.00 × 4, Ala: 1.00 × 2, Cys: small peak, Met: 0.64, Ile: 0.96, Leu: 0.99 Tyr: 0.86, Phe: 0.98 × 2
Elementary analysis
Found:     C 57.62%,  H 6.10%,  N 12.03%
Calculated:  C 57.66%,  H 6.12%,  N 12.26%,
(for C$_{181}$H$_{225}$O$_{38}$N$_{36}$S$_6$Cl$_2\cdot$2H$_2$O)

(6) Synthesis of [D-Ala$^9$]-α-hANP (7—28)

The protected peptide (485 mg, 0.13 mmole) was treated with HF (7 ml) in the presence of anisole (1.25 ml) at −1°C for 60 minutes. The excess HF was distilled off and the residue thus obtained was dissolved in 1N AcOH, and the solution was washed 3 times with Et$_2$O, and passed through a column of the resin "Dowex 1 × 2" (AcO$^-$, 50 ml). The column was eluted with 1N AcOH. The eluent obtained was freeze-dried. The 2SH derivative thus obtained was cyclized by a conventional method to give the disulphide derivative in crude form. This derivative was purified by (1) CM-Cellulose column chromatography 0.05M (pH 4.7) → 0.5M (pH 4.8) NH$_4$OAc (2) "HP-20" column chromatography (0 → 28% CH$_3$CN/5% AcOH), and (3) Sephadex "LH-20" column chromatography (0.5N AcOH) to obtain the required purified product (23 mg).

28

Amino acid analysis
Arg: 1.00 × 3, Asp: 1.02 × 2, Ser: 0.92 × 2, Glu: 1.00, Gly: 1.03 × Ala: 1.00 × 2, Cys: 0.82 × 2, Met: 0.83, Ile: 0.95, Leu: 0.99, Tyr: 0.95, PHe: 1.02 × 2.
Elementary analysis
Found:       C 46.41%,   H 6.15%,   N 16.73%
Calculated:  C 46.26%,   H 6.84%,   N 16.96%
(as $C_{101}H_{155}O_{30}N_{33}S_3 \cdot 2AcOH \cdot 11H_2O$)

## Example 15

Synthesis of [Des Gly$^9$]-α-hANP (7—28)
(1) Synthesis of Boc-Phe-Gly-OBzl
Boc-Phe-OH (79.6 g, 0.3 mole), Gly-OBzl-ToSOH (111 g, 1.1 eq) and HOBt (44.6 g, 1.1 eq) were dissolved in DMF (300 ml), WSCI (60.4 ml, 1.1 eq) was added dropwise thereto with cooling, and the solution was stirred overnight. DMF was distilled off, the residue was distilled in $CHCl_3$, and the solution was washed with 5% $NaHCO_3$, 10% $Na_2O_3$, $H_2O$, 1N HCl, and $H_2O$ in the order give, and dried over $MgSO_4$. The $CHCl_3$ was distilled off and to the residue $Et_2O$ was added. The powder thus produced was recovered by filtration and re-crystallized twice from AcOEt/$Et_2O$ to obtain the required product (59 g).

(2) Synthesis of Boc-Phe-Gly-OH
Boc-Phe-Gly-OBzl (6.2 g, 15 mmole) was suspended in a mixture of MeOH (80 ml) and AcOH (30 ml), and then $H_2$ was passed through the solution in the presence of Pd-C for 4 hours. The Pd-C was removed by filtration, and the solvent was distilled off. The residue thus obtained was recrystallized twice from AcOEt/ n-hexane to give the required product (4.49 g, 92.8%)

(3) Synthesis of Z-Cys(4-$CH_3$Bzl)-Phe-Gly-OH
Boc-Phe-Gly-OH (1.29 g, 4 mmole) was stirred with TFA (10 ml) for 10 minutes with cooling and for 25 minutes at room temperature, and TFA was distilled off therefrom. The residue was treated with $Et_2O$-n-hexane. The powder thus produced was recovered by filtration, dried for 2.5 hours over NaOH, dissolved in DMF (9 ml), and then neutralized with $NEt_3$ with cooling. Z-Cys-(4-$CH_3$Bzl)-OSu (2.1 g, 1.15 eq) was added thereto, and the solution was stirred for 20 hours. AcOEt (50 ml) was added thereto and the solution was washed with 1N HCl and $H_2O$ and then dried over $Na_2SO_4$. AcOEt was distilled off and the residue was treated twice with AcOEt, MeOH, $Et_2O$ and n-hexane to give a powder of the required product (1.5 g, 66.7%).
Amino acid analysis
Gly: 1.00, Cys: small peak, Phe: 0.99
Elementary analysis
Found:       C 63.89%,   H 6.05%,   N 7.46%
Calculated:  C 63.92%,   H 5.90%,   N 7.45%
(as $C_{30}H_{33}O_6N_3S$)

(4) Synthesis of Protected [Des Gly$^9$]-α-hANP (7—28)
Boc-Arg(Tos)-Met-Asp(OChx)-Arg(Tos)-Ile-Gly-Ala-Gln-Ser(Bzl)-Gly-Leu-Gly-Cys(4$CH_3$Bzl)-Asn-Ser(Bzl)-Phe-Arg(Tos)-Tyr(ClBzl)-OBzl (644 mg, 0.2 mmole) was treated with TFA (5 ml) for 10 minutes with cooling and for 40 minutes at room temperature. 5.0N HCl/dioxane (0.1 ml) was added thereto, and the solvent was distilled off. $Et_2O$ was added thereto and the powder thus produced was recovered by filtration and dried overnight over NaOH.
The powder, together with Z-Cys(4-$CH_3$Bzl)-Phe-Gly-OH (118 mg, 1.05 eq) and HOBt (30 mg, 1.1 eq) was dissolved in NMP (8 ml), WSCI (41 microlitres, 1.1 eq) was added thereto with cooling, and the mixture was stirred overnight. To the gelatinized solution, which had a negative response in a fluorescamine test, water was added. The solution was filtered. The solid material thus obtained was washed with water and $Et_2O$, suspended in DMF, and then MeOH was added. The solution was filtered and the solid material thus obtained was washed with MeOH to give the required Product (640 mg, 87.3%).
Amino acid analysis
Art: 1.00 × 3, Asp: 1.00 × 2, Ser: 0.90 × 2, Glu: 1.05, Gly: 1.00 × 4, Ala: 1.00, Cys; small peak, Met: 0.58, Ile: 0.97, Leu: 1.01, Tyr: 0.80, Phe: 0.95 × 2.
Elementary analysis
Found:       C 57.72%,   H 6.46%,   N 11.64%
Calculated:  C 57.51%,   H 6.13%,   N 12.06%

(5) Synthesis of [Des Gly$^9$]-α-hANP (7—28)
The required purified product (30.7 mg) was obtained in the same manner as in Example 14 (6) using as a starting material the protected peptide produced in the above step (5) (476 mg, 10.13 mmole).

Amino acid analysis

Arg: 1.01 × 3, Asp: 1.00 × 2, Ser: 0.90 × 2, Glu: 0.97, Gly: 11.01 × 4, Ala: 1.01, Cys: 0.86 × 2, Met: 0.81, Ile: 0.94, Leu: 0.99, Tyr: 0.95, Phe: 1.01 × 2.

Elementary analysis

Found:  C 46.21%,  H 6.58%,  N 16.86%

Calculated:  C 46.46%,  H 6.80%,  N 17.00%

(as $C_{98}H_{150}O_{29}N_{32}S_3 \cdot 2AcOH.10H_2O$)

## Example 16

Synthesis of (D-Asp$^{13}$]-α-hANP (7—28)

(1) Synthesis of Boc-D-Asp(OBzl)-Arg(Tos)-Ile-Gly-OPac

Boc-Arg(Tos)-Ile-Gly-OPac (1 g, 1.39 mmole) was stirred with TFA (10 ml) for 50 minutes. TFA was distilled off and to the residue, 5.9N HCl/dioxane (0.35 ml, 2.1 mmole) was added thereto, and stirred well. Further, Et$_2$O was added thereto. The precipitate thus produced was recovered by filtration, dried, and dissolved in DMF (8 ml). HOBt (216 mg, 1.6 mmole), Boc-D-Asp(OBzl)-OH (517 mg, 1.6 mmole) and WSCl (0.29 ml, 1.6 mmole) were added to the solution with cooling to −15°C. The solution was stirred for 3 hours to complete the reaction, and water was added. The precipitate thus produced was recovered by filtration and reprecipitated from MeOH/Et$_2$O to obtain the required product (1.1 g, 86.6%).

(2) Synthesis of Boc-Met-D-Asp(OBzl)-Arg(Tos)-Ile-Gly-OPac

A mixture of Boc-D-Asp(OBzl)-Arg(Tos)-Ile-Gly-OPac (1 g, 1.08 mmole) and TFA (10 ml) was stirred for 50 minutes. TFA was distilled off, and to the residue 5.9N HCl/dioxane (0.28 ml, 1.62 mmole) was added and stirred well. Et$_2$O was added thereto. The precipitate thus produced was recovered by filtration, dried, and then dissolved in DMF (10 ml). Thereafter, HOBt (162 mg, 1.2 mmole), Boc-Met-OH (299 mg, 1.2 mmole) and WSCl (0.22 ml, 1.2 mmole) were added thereto with cooling to −15°C. The solution was stirred for 5 hours and water was added to the solution. The precipitate thus produced was recovered by filtration and re-precipitated from MeOH/Et$_2$O to obtain the required product (960 mg, 84.2%).

(3) Synthesis of Boc-Arg(Tos)-Met-D-Asp(OBzl)-Arg(Tos)-Ile-Gly-OPac

The required product (750 mg, 64.7%) was obtained in the same manner as in the above step (2), using as starting materials Boc-Met-D-Asp(OBzl)-Arg(Tos)-Ile-Gly-OPac (0.9 g, 0.85 mmole) and Boc-Arg(Tos)-OH (471 mg, 1.1 mmole).

(4) Synthesis of Boc-Cys(4-CH$_3$Bzl)-Phe-Gly-Gly-Arg(Tos)-Met-D-Asp(OBzl)-Arg(Tos)-Ile-Gly-OPac

A mixture of Boc-Arg(Tos)-Met-D-Asp(OBzl)-Arg(Tos)-Ile-Gly-OPac (650 mg, 0.48 mmole) and TFA (5 ml) was stirred for 50 minutes. TFA was distilled off and to the residue, 5N HCl/dioxane (0.14 ml, 0.72 mmole) was added, and stirred well. Et$_2$O was added thereto. The precipitate thus produced was recovered by filtration, dried and dissolved in DMF (5 ml). Then, HOBt (72 mg, 0.53 mmole), Boc-Cys(Bzl)-Phe-Gly-Gly-OH (311 mg, 0.53 mmole) and WSCl (0.1 ml, 0.53 mmole) were added thereto with cooling to −15°C, and the solution was then stirred for 4 hours. To the reaction, water was added. The precipitate thus produced was recovered by filtration and reprecipitated from MeOH to obtain the required product (720 mg, 81.8%).

(5) Synthesis of Boc-Cys(4-CH$_3$Bzl)-Phe-Gly-Gly-Arg(Tos)-Met-D-Asp(OBzl)-Arg(Tos)-Ile-Gly-OH

The peptide as produced above in step (4) (690 mg, 0.37 mmole) was dissolved in AcOH (40 ml) and the solution was stirred for 1 hour in the presence of zinc powder (1 g) at 45°C. The zinc powder was removed by filtration, and AcOH was distilled off. To the residue water was added and the precipitate thus produced was recovered by filtration and recrystallized from MeOH to obtain the required product (500 mg, 78.9%).

(6) Synthesis of Protected [D-Asp$^{13}$]-α-hANP (7—28)

Boc-Cys(4-CH$_3$Bzl)-Phe-Gly-Gly-Arg(Tos)-Met-D-Asp(OBzl)-Arg(Tos)-Ile-Gly-Ala-Gln-Ser(Bzl)-Gly-Leu-Gly-Cys(4-CH$_3$Bzl)-Asn-Ser(Bzl)-Phe-Arg(Tos)-Tyr(ClBzl)-OBzl

A mixture of Boc-Ala-Gln-Ser(Bzl)-Gly-Leu-Gly-Cys(4-CH$_3$Bzl)-Asn-Ser(Bzl)-Phe-Arg(Tos)-Tyr(Cl$_2$Bzl)-OBzl (170 mg, 0.081 mmole) and TFA (5 ml) was stirred for 50 minutes. TFA was distilled off and to the residue 5.0N HCl/dioxane (3.0 microlitres, 0.13 mmole) was added, and stirred well. Et$_2$O was added thereto. The precipitate thus produced was recovered by filtration and dissolved in NMP (8 ml) and HOBt (13.5 mg, 0.1 mmole) and the peptide as obtained above in step (5) (160 mg, 0.1 mmole) were added thereto with cooling to −15°C. The solution was stirred for 16 hours and then water was added to the reaction solution. The precipitate thus produced was recovered by filtration and refluxed twice with MeOH to obtained the required product (280 mg, 94.5%).

(7) Synthesis of [D-Asp$^{13}$]-α-hANP (7—28)

A mixture of the protected [D-Asp$^{13}$]-α-hANP (7—28) (270 mg, 0.073 mmole) and TFA (5 ml) was stirred for 50 minutes. TFA was distilled off and to the residue ET$_2$O was added. The precipitate thus produced was recovered by filtration and dried over NaOH in a desiccator for 16 hours. To the precipitate, anisole (0.5 ml) and HF (5 ml) were added, and the solution was stirred to cause reaction for 60 minutes at −1°C. HF was distilled off and to the residue, Et$_2$O was added. The precipitate thus produced was obtained by extraction with 2N AcOH. It was washed with Et$_2$O and passed through a column of the resin "Dowex 1 × 2" (AcO⁻), and the eluent obtained was freeze-dried. All of the thus obtained powder was dissolved in 1N AcOH (8 ml) and the solution was added dropwise to a mixture of 1M NH$_4$OAc/8M urea (72 ml) and K$_3$Fe(CN)$_6$ (34 mg, 0.102 mmole) over 20 minutes. The pH of the reaction solution was kept at 7.4 with 10% NH$_4$OH. Thereafter, the reaction solution was adjusted to pH 4.5 with AcOH. Next, the solution was passed through a column of the resin "IRA-45" (Cl⁻ 50 ml) and was washed with 1N AcOH. The washing liquid was desalted in a column of the resin "Diaion HP-20" and the eluent obtained was freeze-dried. All of the obtained powder was purified by CM-cellulose column chromatography using gradient elution and using 0.05M NH$_4$OAc (pH 5) (500 ml) → 0.4M NH$_4$OAc (pH 6) (500 ml). The fractions 35 to 42 were collected, combined together and freeze-dried. The product was purified by "Diaion HP-20" column chromatography (elution with 5% → 25% CH$_3$CN/5% AcOH). The fractions 75 to 87 were collected, combined together and freeze-dried. The powder thus obtained was dissolved in 1N AcOH and passed through a column of the resin "Dowex 1 × 2" (AcO⁻), and the eluent obtained was freeze-dried.

The powder thus obtained was desalted by a column of the resin "LH-20", and eluted with 1N AcOH. The eluent obtained was freeze-dried to obtain the required product (21 mg).

Amino acid analysis

NH$_3$: 2.66, Arg 1.03 × 3, Asp: 1.00 × 2, Ser: 0.89 × 2, Glu: 0.96, Gly: 1.01 × 5, Ala: 1.00, 1/2(Cys)$_2$: 0.83 × 2, Met: 0.85, Ile: 0.95, Leu: 0.97, Tyr: 0.95, Phe: 1.00 × 2.

HPLC

Column: Nucleosil 5C$_{18}$; Eluting Solvent: 25% CH$_3$CN/0.1% TFA).

Time: 62 minutes

## Example 17

Diuretic Tests

Diuretic tests were carried out on the peptide produced in the above examples. The results obtained are given in the following Table.

EP 0 173 557 B1

### Table (Test of Pharmacological Activity)

| Compound (Example No.) | Spasmolytı Activity in Rat Aorta (a) | Spasmolytic Activity in Chick Rectum (a) | Natriuretic Activity in Anesthetized Rat |
|---|---|---|---|
| $\alpha$-hANP(1-28) (Control) | 100 | 100 | ++ |
| " (5-28) (Ex.2) | $110^{\pm}7.6(5)$ | $73.6^{\pm}1.9(2)$ | + |
| " (7-28) (Ex.5) | $148^{\pm}24.5(6)$ | $443^{\pm}7.5(2)$ | +++ |
| " (5-27) (Ex.1) | $103^{\pm}25.5(4)$ | $60.3^{\pm}5.1(2)$ | +++ |
| " (5-25) (Ex.4) | $6.67^{\pm}1.23(4)$ | $30.7^{\pm}3.6(6)$ | + |
| [Nle$^{12}$]-$\alpha$-hANP(1-28) (Ex.3) | $80.5^{\pm}12.2(4)$ | $70.3^{\pm}10.9(2)$ | |
| [Met(O)$^{12}$]-$\alpha$-hANP(1-28) (Ex.7) | $4.72^{\pm}0.70(4)$ | $2.89^{\pm}0.67(4)$ | |
| [Asu$^{7,23}$]-$\alpha$-hANP(7-23) (Ex.6) | $1.03^{\pm}0.48(3)$ | $105^{\pm}18(4)$ | + |
| [Met$^{12}$,Asu$^{7,23}$]-$\alpha$-hANP(7-28)(Ex.8) | $10.9^{\pm}0.65(6)$ | $82^{\pm}8.3(4)$ | + |
| [Ile$^{12}$,Asu$^{7,23}$]-$\alpha$-hANP(7-28)(Ex.9) | $124^{\pm}4.2(2)$ | $90.9^{\pm}3.0(6)$ | +++ |
| [Nle$^{12}$,Asu$^{7,23}$]-$\alpha$-hANP(7-28)(Ex.10) | $7.5^{\pm}0.9(2)$ | $37.6^{\pm}4.5(2)$ | + |
| [Nle$^{12}$]-$\alpha$-hANP(7-28) (Ex.11) | $57^{\pm}3(4)$ | $189^{\pm}10(2)$ | +++ |
| $\alpha$-hANP(4-28) (Ex.12) | $61^{\pm}3(2)$ | $56^{\pm}4(2)$ | ++ |
| $\alpha$-hANP(7-27) (Ex.13) | 73.7(2) | 200(5) | ++ |
| [D-Ala$^{9}$]-$\alpha$-hANP(7-28) (Ex.14) | 121(4) | 344(3) | +++ |
| [DesGly$^{9}$]-$\alpha$-hANP(7-28) (Ex.15) | 0.2(2) | 2.3(3) | — |
| [D-Asp$^{13}$]-$\alpha$-hANP(7-28) (Ex.16) | 0.4(2) | 1.9(3) | — |

Values are mean $\pm$ Standard Error; Number of experiment are in parenthesis
(a) : 50% effective dose (ED$_{50}$) for each preparation was obtained and the ratio was calculated to the standard hANP (1-28)

Test methods (assay of the pharmacological activities of ANP)

(1) Spasmolytic activity on rat aortic strips

Male Spraque-Drawley rats weighing 250—280 g were used. After decapitation of the rat, its thoracic aorta was obtained, and aorithic helical strips were prepared. Krebs solution was used in this assay system. The aortic strio was suspended in a 20 ml organ bath which was aerated with 5% carbon dioxide and 95% oxygen and kept at 37 ± 0.5°C. The aortic tension was recorded isotonically. As the isotonic recording system, a transducer and amplifier (ME commercial ME4012) and recorder (Sanei 8K21) were used. A resting tension of 0.5 g was loaded on the aortic strip. The preparation was left under this condition for 1 hour to obtain stable tension. The aorta preparation, contracted with norepinephrine ($5 \times 10^{-8}$M), was exposed to ANP to examine its relaxant activity. The dose responsive curve was obtained for the α-hAN (1—28) and ANP fragment in the same preparation. The 50% effective does (ED50) for each preparation was obtained and the ratio was used as the specific acitivity.

(2) Spasmolytic activity in chick rectum

Male 2—3-week-old chicks weighing 155—175 g were used. After anaesthetizing the chick (60 mg/kg pentobarbital i.p.), its rectum was excised. Krebs solution was used. The rectrum was suspended in a 20 ml organ bath aerated with 5% carbon dioxide and 95% oxygen and kept at 37 ± 0.5°C. The contractil tension of the rectum was measured isotonically. The isotonic recording system used was the same as that used above. The rectum, under 0.5 g of tension, was stabilized by keeping it under this condition for 1 hour. The rectum, contracted with carbachol ($2 \times 10$M), was exposed to ANP to examine its relaxing effect. As with the rat aorta preparation, the ED50 for the standard hANP (1—28) and ANP fragment were determined and the specific activity calculated. The relaxing activity of the sample was examined on the rectum when contracted with Carbocoal ($2 \times 10^{-7}$M).

(3) Natriuretic activity in anesthetized rats

Male Sprague-Dawley rats each weighing 250—300 g were used. After anesthetizing them by intraperitoneal administration of 50 mg/kg of pentobarbital, a cannula was inserted into the trachea to maintain the airway. The blood pressure and heart rate were recorded through a cannula inserted into the fermoral artery. As a recording sytem, a blood pressure transducer (Century Technology, CP-01), amplifier (Star Medical, PA-011), heart rate counter (Star Medical, HR-001), and recorded (Rika Denki, R-302) were used.

Ringel's solution was infused into a cannular inserted into the femoral vein at the rate of 20 microlitres/ minute using a perfusion pump (Ikouseiki M-1V). ANP was administered through their cannula.

A cannula was inserted into the bladder, and a urine sample was collected at 10 minute intervals into a 2 ml sampling tube. The volume of urine was measured by weighing it with an automatic balance (Metler, AE160). The sodium and potassium contents were measured using the 20 microlitres of urine obtained, with a glass electrode ion concentration meter (Orion research 901).

**Claims**

1. The peptide having the formula:

H-Cys-Phe-Gly-Gly-Arg-Met-Asp-Arg-Ile-Gly-Ala-Gln-Ser-
Gly-Leu-Gly-Cys-Asn-Ser-Phe-Arg-Tyr-OH.

2. The peptide having the formula:

Phe-Gly-Gly-Arg-Met-Asp-Arg-Ile-Gly-Ala-Gln-Ser-Gly-
Leu-Gly-Asu-OH.

3. A salt of a peptide as claimed in claim 1 or 2.

4. A pharmaceutical composition comprising (a) a peptide as claimed in any of claims 1 to 3, or a pharmaceutically-acceptable salt thereof, and (b) a pharmaceutically-acceptable carrier or diluent therefor.

# EP 0 173 557 B1

**Patentansprüche**

1. Peptid der Formel:

H-Cys-Phe-Gly-Gly-Arg-Met-Asp-Arg-Ile-Gly-Ala-Gln-Ser-Gly-Leu-Gly-Cys-Asn-Ser-Phe-Arg-Tyr-OH.

2. Peptid der Formel:

Phe-Gly-Gly-Arg-Met-Asp-Arg-Ile-Gly-Ala-Gln-Ser-Gly-

Leu-Gly-Asu-OH.

3. Salz eines Peptids gemäß Anspruch 1 oder 2.

4. Pharmazeutische Zusammensetzung, enthaltend (a) ein Peptid gemäß einem der Ansprüche 1 bis 3 oder ein pharmazeutisch geeignetes Salz davon und (b) ein pharmazeutisch geegnetes Trägermaterial oder Verdünnungsmittel dafür.

**Revendications**

1. Le peptide ayant la formule:

H-Cys-Phe-Gly-Gly-Arg-Met-Asp-Arg-Ile-Gly-Ala-Gln-Ser-Gly-Leu-Gly-Cys-Asn-Ser-Phe-Arg-Tyr-OH.

2. Le peptide ayant la formule:

Phe-Gly-Gly-Arg-Met-Asp-Arg-Ile-Gly-Ala-Gln-Ser-Gly-

Leu-Gly-Asu-OH.

3. Un sel d'un peptide selon la revendication 1 ou 2.

4. Une composition pharmaceutique comprenant (a) un peptide selon l'une quelconque des revendications 1 à 3, ou un sel pharmaceutiquement acceptable de ce dernier, et (b) un support ou diluant pharmaceutiquement acceptable.